# EUROPEAN PATENT SPECIFICATION

(11) **EP 4 531 943 B1**
(45) Date of publication and mention of the grant of the patent: **22.07.2026**
(21) Application number: 23728395.7
(22) Date of filing: 24.05.2023
(51) Int. Cl.: A61L 27/20, A61L 27/38, A61L 27/52, B33Y 70/00, B33Y 80/00

(54) **INK FORMULATION, METHOD OF PRODUCING THE SAME AND USES OF THE INK FORMULATION**
TINTENFORMULIERUNG, VERFAHREN ZU IHRER HERSTELLUNG UND VERWENDUNGEN DER TINTENFORMULIERUNG
FORMULATION D'ENCRE, SA MÉTHODE DE PRODUCTION ET UTILISATIONS DE LA FORMULATION D'ENCRE

(30) Priority: 24.05.2022 FI 20225454
(43) Date of publication of application: 09.04.2025
(73) Proprietor: Åbo Akademi, 20500 Åbo (FI)
(72) Inventor: WANG, Xiaoju, 20500 Åbo (FI); BRUSENTSEV, Yury, 20500 Åbo (FI); XU, Wenyang, 20500 Åbo (FI); XU, Chunlin, 20500 Åbo (FI); WÅGBERG, Lars, 20500 Åbo (FI); BACKMAN, Oskar, 20500 Åbo (FI); WILLFÖR, Stefan, 20500 Åbo (FI)
(74) Representative: Laine IP Oy
(86) International application number: PCT/FI2023/050292
(87) International publication number: WO 2023/227831

(56) References cited:
- CN-B- 110 787 320
- XU WENYANG ET AL: "On Low-Concentration Inks Formulated by Nanocellulose Assisted with Gelatin Methacrylate (GelMA) for 3D Printing toward Wound Healing Application", APPLIED MATERIALS & INTERFACES, vol. 11, no. 9, 6 March 2019 (2019-03-06), US, pages 8838 - 8848, XP055861527, ISSN: 1944-8244, Retrieved from the Internet <URL:https://pubs.acs.org/doi/pdf/10.1021/acsami.8b21268> DOI: 10.1021/acsami.8b21268
- MA QIAN ET AL: "UV-Curable Cellulose Nanofiber-Reinforced Soy Protein Resins for 3D Printing and Conventional Molding", ACS APPLIED POLYMER MATERIALS, vol. 2, no. 11, 9 September 2020 (2020-09-09), pages 4666 - 4676, XP093063409, ISSN: 2637-6105, DOI: 10.1021/acsapm.0c00717
- GILBERTO SIQUEIRA ET AL: "Cellulose Nanocrystal Inks for 3D Printing of Textured Cellular Architectures", ADVANCED FUNCTIONAL MATERIALS, vol. 27, no. 12, 7 February 2017 (2017-02-07), DE, pages 1604619, XP055434086, ISSN: 1616-301X, DOI: 10.1002/adfm.201604619
- WANG XIAOJU ET AL: "Nanocellulose-Based Inks for 3D Bioprinting: Key Aspects in Research Development and Challenging Perspectives in Applications-A Mini Review", BIOENGINEERING, vol. 7, no. 2, 29 April 2020 (2020-04-29), pages 40, XP055949417, DOI: 10.3390/bioengineering7020040

## Description

### FIELD

The present invention relates to ink formulations based on methacrylated cellulose nanofibrils. In particular, the present invention concerns ink formulations for 3D printing, comprising a hydrogel containing methacrylated cellulose nanofibrils and preferably a cross-linking agent, optionally together with biological material, methods of producing such ink formulations, as well as uses thereof for, e.g., preparing 3D hydrogel scaffolds for cell culture and drug screening.

### BACKGROUND

The development of novel fabrication processes such as three-dimensional (3D) additive manufacturing, known as 3D printing, provides untapped sustainable means to tailor bio-based functional materials towards high value applications in e.g. biomedical areas. Biomedical hydrogels are one intriguing profiling area for nanocelluloses.

For developing hydrogel scaffolds that mimic the three-dimensional (3D) architecture of tissue and recapitulate biological function, 3D bioprinting outstands to enable creating individually tailor-made tissue engineering scaffolds to provide desired architecture, and furthermore, integrating with biological cues to direct cell response in a controlled manner.

Recently, nanocelluloses have emerged as renewable constituents in formulating bioinks, which are extrudable thanks to the shear-thinning rheological properties. One of the challenges to apply the nanocellulose-based bioink in 3D bioprinting lies on the means to keep ink fidelity after the extrusion and further to achieve mechanical integrity of the prints during and after printing.

Some approaches to tune the mechanical strength of printed scaffolds have been developed. For example, cellulose nanofibrils (CNFs) have been crosslinked during printing by addition of aqueous Ca²⁺ solution, followed by a post-printing chemical crosslinking with 1,4-butanediol diglycidyl ether. With further tuning the crosslinking parameters, the mechanical strength (compressive Young's modulus) of the printed CNF scaffolds was achieved in the range of 3 to 8 kPa (Xu, C. et al., 2018).

In another approach, a low-concentration ink formulation based on 1 w/v% CNF and up to 1 w/v% of gelatin methacrylate (GelMA) was developed. By direct ink writing technique assisted by UV post-curing, high-resolution scaffolds of CNF/GelMA were printed and demonstrated high fidelity. By tuning the compositional ratio between CNF and GelMA, the compressive Young's modulus and local surface stiffness could be tuned (Xu, W. et al., 2019a).

In a further attempt, methacrylate of galactoglucomannan (GGM), a wood biopolymer, was synthesized and used as an auxiliary component with CNFs in the ink formulations. By tuning the substitution degree of galactoglucomannan methacrylate (GGMMA) and compositional ratio between CNF and GGMMA, the compressive Young's moduli of the final scaffolds presented a tunable wide spectrum from 2.5 to 22.5 kPa (Xu W. et al., 2019b).

In the above approaches nanocellulose, which was prepared by TEMPO-medicated oxidation and possessed high charge density, was used as such in the ink formulations.

Recently, Ma et al (2020) reported the synthesis of methacrylated cellulose nanofibrils (CNF) by reacting the CNF suspended from dry powder in water with methacrylic anhydride and further utilized the methacrylated CNF to reinforce a UV-curable soy protein resin in direct molding and laser printing. Also in CN 110787320 B and US 2021171773 A1 a dry powder of CNFs is used as starting material. However, there was no quantitative analysis on the substitution degree of methacryloyl groups available to reflect the efficiency of the product in photo-crosslinking. Such an approach adding methacrylic anhydride into suspension of CNF is expected to result in extreme low degree of methacrylation, very often not sufficient enough to be quantified in the current available characterization tools. Thus, direct proof of methacrylation on CNF has remained not possible to present.

There exists a need for ink formulations free of cells as well as bioink formulations that can form stable hydrogel or dispersion with cells in culture media. The inks should provide an opportunity to design 3D hydrogel scaffolds with controlled mechanical strength, viscoelasticity and hydration, as well as with excellent ink fidelity and workability in terms of filament resolution, construct integrity, and geometry complexity of the hydrogel constructs. Meanwhile, cellular compatibility of the biomaterials is a primary request for the selection of formulation components in order for the bioinks to support the adhesion, proliferation, migration and differentiation of living cells.

### SUMMARY OF THE INVENTION

The invention is defined by the features of the independent claims. Some specific embodiments are defined in the dependent claims.

It is one of the aims of the present invention to eliminate at least a part of the problems relating to the art and to provide novel bioink formulations with the above mentioned advantageous properties.

The present invention is based on the concept of providing an ink formulation which comprises non-charged to medium-charged cellulose nanofibrils with methacrylate derivatization and preferably cross-linking polymers as a basis of ink formulations. The cross-linking polymers may be derived from bio-based resources and incorporated with UV-crosslinkable moieties such as methacrylate. The methacrylates on both components may be crosslinked upon UV treatment. The ink formulations of the invention may be printed into scaffolds for example for cell culture and drug screening. The present invention thus provides methacrylated CNF-based photo-crosslinkable ink formulations, with a sufficient degree of substitution (DS) of MA on the cellulose nanofibrils, which result in highly dispersible CNF hydrogels.

According to a first aspect of the present invention, there is provided a hydrogel formulation, comprising methacrylated cellulose nanofibrils, optionally a crosslinking agent, and water, wherein the cellulose nanofibrils have a charge density of 1.2 mmol/g or lower, preferably less than 1.2 mmol/g, and wherein the methacrylated cellulose nanofibrils comprise cellulose nanofibrils obtained from cellulose fibers by treatments that introduce anionic charges.

According to a second aspect of the present invention, there is provided a method of producing an ink formulation for 3D printing, comprising the steps of providing an aqueous gel of methacrylated cellulose nanofibrils, preferably providing a photoinitiator, optionally providing at least one additional crosslinking agent, optionally providing an aqueous dispersion of biological material, and mixing the gel, the optional crosslinking agent and optionally the aqueous dispersion of biological material to form an ink composition, wherein the cellulose nanofibrils have a charge density of 1.2 mmol/g or lower and comprise methacrylated cellulose nanofibrils obtained from cellulose fibers subjected to oxidation in an aqueous medium.

One embodiment of the invention comprises a method of producing the aqueous gel of methacrylated cellulose nanofibrils, wherein the method comprises the steps of oxidizing cellulose fibers in an aqueous medium, subjecting the oxidized cellulose to solvent exchange from water to an organic solvent, preferably to dimethylformamide, surface modification of hydroxyl groups on cellulose fibers with acrylic anhydrides , such as methacrylic anhydride or acrylic anhydride, removal of the organic solvent and mechanical defibrillation of the methacrylated cellulose fibers in water to obtain the aqueous gel of methacrylated cellulose nanofibrils. The cellulose nanofibrils remain undried from water or organic solvent throughout the process of above-mentioned modifications.

Further embodiments of the invention comprise the use of the ink formulation in 3D printing, for example to verify the work ability of a 3D bioprinter, to design 3D hydrogel scaffolds as cell culture platforms for cell proliferation and cell differentiation, e.g. cancer cells, for cell-matrix and cell-cell interaction studies and for drug screening or drug therapy studies. Another embodiment of the invention comprises the hydrogel formulation for use in tissue and organ engineering and regenerative medicine.

The invention also relates to 3D printed products comprising the hydrogel formulation (ink formulation) according to the present invention.

Considerable advantages are obtained by the invention. The ink formulations allow to tune the mechanical strength, hydration and viscoelasticity of scaffolds to a broad window and to design 3D hydrogel scaffolds with controlled mechanical stiffness. A broad stiffness window will find applications for cell culture or tissue engineering of wide range of cell lines and tissues, respectively. The ink formulations of the invention are highly transparent and provide a high biocompatibility and cytocompatibility as well as high resolution and stability of the printed objects. The formed 3D matrix is highly porous better allowing cells to migrate and proliferate.

Second, the ink formulation may be formulated together with cells into a bioink formulation that can form a stable hydrogel or stable 3D printed cell-laden constructs or dispersion with cells in culture media. The ink formulation contains non-charged to medium-charged cellulose nanofibrils, which enables to make inks that can form stable phase with cells in their typically high ionic strength media.

Further, high stability is allowed in several aspects including 1) highly stable as ready-to-go bioink formulations (not causing phase separation) to provide an easy flow during printing, and keeping its structure after printing; 2) In some embodiments, in highlight of the intrinsic affinity of hemicellulose anchoring onto cellulose surface, it was discovered that hemicellulose, here for example in the form of GGMMA (methacrylated galactoglucomannan), may interact with CNFMA in formulation and as result, the viscosity of dispersion with the addition of GGMMA is reduced in comparison to that of CNFMA. Such a feature provides a better flow ability during printing and allows lower pressure and less stress to the cells while printing. Therefore, the cells that are formulated and used in the present bioinks, in particular in the embodiments comprising a methacrylated biopolymer, encounter less stress and thus are less damaged. 3) Each printed layer will be kept more intact and not flowing away during printing as well as keeping well the constructs without fading away by crosslinking the methacrylate groups via shedding visible lights during and after printing. The 3D printed scaffolds will also be highly stable due to the provided high crosslinking efficiency.

Further features and advantages of the present technology will appear from the following description of some embodiments.

### BRIEF DESCRIPTION OF THE DRAWINGS

FIGURE 1. Fig. 1a) illustrates the surface modification on fiber with methacrylic anhydride in DMF, b) AFM visualization of fibrils of TEMPO-CNF and CNF-MA; c) TEM images of TEMPO-CNF and CNF-MA in accordance with at least some embodiments of the present invention;

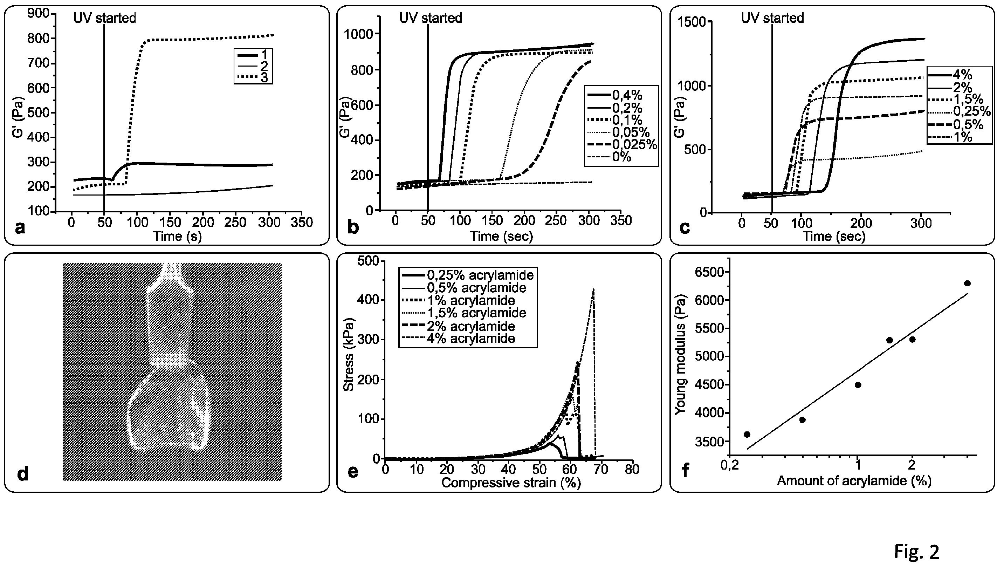

FIGURE 2(a): Photo-rheology time sweep. 1. CNF-MA (1.1%), Irgacure 2959 (0.4%); 2. n/m-CNF (1.1%), Acrylamide (1%), Irgacure 2959 (0.4%); 3. CNF-MA (1.1%), Acrylamide (1%), Irgacure 2959 (0.4%). FIG 2(b): Photo-rheology time sweep CNF-MA (1.1%), Acrylamide (1%), variation of Irgacure 2959 (0-0.4%). FIG 2(c): Photo-rheology time sweep CNF-MA (1.1%), Irgacure 2959 (0.2%), variation of Acrylamide (0.25-4%). FIG 2(d): Disc of the cross-linked material prepared in the rheometer during the photo-rheology experiment. FIG 2(e): Compression curves for the cross-linked material containing CNF-MA (1.1%), Irgacure 2959 (0.2%) and variation of Acrylamide (0.25-4%) FIG 2(f): Linear approximation of the Young modulus (average value) dependence on the logarithm of acrylamide concentration.
FIGURE 3 illustrates swelling behaviour of aerogels of photo polymerized CNF-MA+AA via different drying procedures and SEM images of the cross-sections of the CNF-MA crosslinked with polyacrylamide and n/m-CNF Ca2+ cross-linked material.
FIGURE 4 shows comparison of the Young's modulus (a) and compression curves (b) for the 1.1% CNF-MA+1% PAA hydrogels and 1.1% CNF-MA crosslinked by CaCl₂.

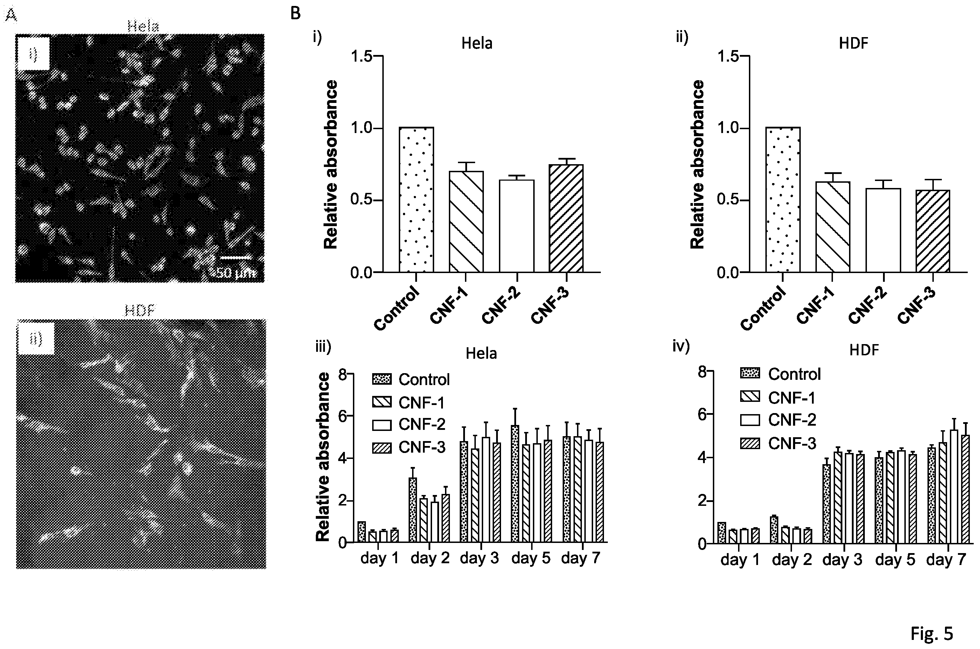

FIGURE 5A. Representative confocal images of the cells were recorded after 48 of incubation for Hela (i) and HDF (ii) in the 24-well with the glass-bottom plates pre-coated with different CNF-MA hydrogels. The cell morphology was shown by Phalloidin (green) and nuclei were counterstained by DAPI (blue). Scale bar: 50 µm. FIG 5B. Cell survival rates on the plates pre-coated with different CNF-MA hydrogels were measured for Hela (i) and HDF (ii) in a density of 5000 cells/well by using cytotoxicity assay. Cell proliferation rates on the plates pre-coated with different CNF-MA hydrogels were for Hela (iii) and HDF (iv) in a density of 5000 cells/well by using cytotoxicity assay for incubation of 1, 2, 3, 5, and 7 days. CNF-1, -2, and -3 are three types of CNF-MA+PAA hydrogels cross-linked from 1.1% CNF-MA and acrylamide of varied concentration of 0.25%, 0.5%, and 1%, respectively.
FIGURE 6 (A) Extrusion of filament using 1.1% CNF-MA + 1% acrylamide under variant pressure in a pneumatic dispensing system and conical polyethylene nozzle (250 µm) with a printing speed of 600 mm/min. FIG 6(B) Complex scaffolds 3D printed using pneumatic extrusion. FIG 6(C) Evaluation of the resolution of 3D printing utilizing screw dispensing system.
FIGURE 7. Photo-rheology time sweep. FIG. 7 is a figure on photorheology of formulations of CNF and GGMMA vs. CNF-MA and GGMMA. Full run of 300 sec, linear Y-axis.

### EMBODIMENTS

### DEFINITIONS

In general, "bioink" refers to a multicomponent biomaterial formulation or composition that can be used in 3D bioprinting to achieve a matrix, in particular a biocompatible matrix, which may provide appropriate surface and adequate spaces to accommodate cells and other bioactive substances as well to foster and direct the crucial cellular activities in three dimensions. In the present context, the term "bioink" or "bioink formulation" typically comprises an ink formulation together with biological material, while "ink formulation" or "hydrogel formulation" refer to the formulation without biological material.

In the present context, "hydrogels" stand for a colloidal gel in which water is the dispersion medium and wherein polymer chains and/or nano-dimensional fibrils form a three-dimensional, typically hydrophilic network, which entraps the water.

Within this disclosure, the terms "nanocellulose" and "nanocellulose component" stand in particular for cellulose nanofibers or microfibers or, preferably, cellulose nanofibrils (CNF) which are also referred to as nanofibrillated cellulose (NFC) or microfibrillated cellulose (MFC). The nanocellulose can also be bacterial nanocellulose, i.e. nano-structured cellulose produced by bacteria.

Typically, the nanocellulose exhibits fibrils having a fibril diameter of about 5 to 60 nm, typically about 5 to 20 nm, with a length of up to 25 µm. The aspect ratio for the nanocellulose fibrils ranges from 1 to 10,000, in particular 10 to 5,000, for example about 20 to 1500.

In one embodiment, the present ink formulation comprises at least the following components, mixed with each other:
- methacrylated cellulose nanofibrils, wherein the cellulose nanofibrils have a charge density of 1.2 mmol/g or lower,
- preferably at least one crosslinking agent, particularly a photoinitiator, and
- water.

In one embodiment, the present ink formulation comprises at least the following components, mixed with each other:
- methacrylated cellulose nanofibrils, wherein the cellulose nanofibrils have a charge density of 1.2 mmol/g or lower,
- a water-soluble photoinitiator,
- preferably at least one additional crosslinking agent, and
- water.

The ink formulation comprising the methacrylated cellulose nanofibrils, water and preferably at least one crosslinking agent form a hydrogel.

The ink formulation is capable of 3D printing, for example using an extrusion based 3D printer. In one embodiment, the ink formulation is capable of 3D printing using a direct ink writing (DIW)-type printer.

In one embodiment, the present ink formulation is used for preparing a bioink formulation. Such a formulation can be obtained by admixing a composition, in particular an aqueous composition, such as an aqueous suspension, of biological material with the ink formulation comprising methacrylated cellulose nanofibrils.

Thus, in one embodiment, the present bioink formulation comprises a hydrogel containing at least the following components, mixed with each other:
- methacrylated cellulose nanofibrils, wherein the cellulose nanofibrils have a charge density of 1.2 mmol/g or lower,
- preferably at least one crosslinking agent, and
- biological material.

In one embodiment, the present bioink formulation comprises a hydrogel containing at least the following components, mixed with each other:
- methacrylated cellulose nanofibrils, wherein the cellulose nanofibrils have a charge density of 1.2 mmol/g or lower,
- at least a photoinitiator, preferably with at least one additional crosslinking agent, and
- biological material.

The dispersion medium of the hydrogel is water which forms the aqueous phase of the hydrogel. In addition to the above-mentioned components, the ink formulation may contain further components, such as biocompatible additional components, for example biocompatible rheology modifiers and photoinitiators.

In one embodiment, the formulation comprises 0.1 to 3 %, in particular 0.5 to 2 %, of methacrylated cellulose nanofibrils, 0.1 to 5 %, in particular 0.5 to 3 %, of a crosslinking agent, calculated from the total weight of the formulation. Typically, the rest of the formulation comprises water. The water content of the formulation may thus be at least 92 %, preferably at least 95 %, based on the total weight of the formulation.

### Cellulose nanofibrils

In embodiments, non-charged to medium-charged cellulose nanofibrils, in particular cellulose nanofibrils having a charge density of 1.2 mmol/g or lower, preferably less than 1.2 mmol/g, more preferably less than 0.8 mmol/g, are applied together with crosslinking agents, preferably UV crosslinking agents, in the formulation. In embodiments, non-charged to medium-charged cellulose nanofibrils, in particular cellulose nanofibrils having a charge density of 1.2 mmol/g or lower, preferably less than 1.2 mmol/g, more preferably less than 0.8 mmol/g, are applied, together with a photoinitiator and preferably together with additional crosslinking agents, preferably UV crosslinking agents, in the formulation. Such a non-charged to medium-charged nanocellulose will allow the formulation of bioinks where cells and cell media can be added but do not cause phase separation prior to 3D printing.

In one embodiment, the cellulose nanofibrils have a charge density of less than 0.8 mmol/g, in particular less than 0.5 mmol/g, suitably less than 0.4 mmol/g, such as less than 0.3 mmol/g.

In one embodiment, the cellulose nanofibrils are free of charge or essentially free of charge or free of additional charge by chemical or biochemical modification. In the present disclosure, when the cellulose nanofibrils are essentially free of charge, they have no detectable charge when measured by titration or against pH adjustment by acid-alkali titration.

The charge density of the cellulose nanofibrils can be determined by titration, for example from dispersions of the nanocellulose fibrils. In one embodiment, the conductivity of a water dispersion of nanocellulose fibrils is measured against pH adjustment by acid-alkali titration.

In one embodiment, the nanocellulose hydrogel comprises cellulose nanofibrils obtained from cellulose fibers selected from the group of dissolving pulp, cotton fiber, bacterial cellulose fiber, and combinations thereof, by mechanical defibrillation, such as grinding,

Cellulose nanofibers comprise cellulose nanofibrils (CNFs) or nanofibrillated cellulose (NFC) or combinations thereof.

According to the invention, the cellulose nanofibrils are obtained from cellulose fibers by treatments that introduce anionic charges, e.g. by carboxymethylation, oxidation, TEMPO-oxidation, or acid treatment using sulfuric acid, hydrochloric acid, or phosphoric acid, or a combination thereof.

In one embodiment, the cellulose nanofibrils comprise cellulose nanofibrils obtained by removal of charge groups from cellulose nanofibrils having a charge of at least 1.2 mmol/g to produce cellulose nanofibrils having a charge density of less than 1.2 mmol/g, in particular less than 0.8 mmol/g, such as less than 0.5 mmol/g, suitably less than 0.4 mmol/g, such as less than 0.3 mmol/g.

In one embodiment, the cellulose nanofibrils comprise cellulose fibers subjected to oxidation in aqueous medium, followed by surface modification of hydroxyl groups on cellulose fibers with methacrylic anhydride or other anhydrides with alkenes (such as acrylic anhydride) and mechanical defibrillation of the methacrylated cellulose fibers to methacrylated cellulose nanofibrils. In a further embodiment, the oxidized cellulose is subjected to solvent exchange from water to an organic solvent, preferably to dimethylformamide, followed by surface modification of hydroxyl groups on cellulose fibers with methacrylic anhydride, and removal of the organic solvent and mechanical defibrillation of the methacrylated cellulose fibers to methacrylated cellulose nanofibrils. In this embodiment, methacrylation of cellulose fibers thus takes place on the hydroxy groups of the cellulose fibers. Therefore, the methacrylated cellulose nanofibrils comprise cellulose nanofibrils methacrylated on the OH groups of cellulose fibers.

Apart from the abovementioned reaction route with methacrylic anhydride, methacrylation of cellulose fibers can be carried out on the hydroxyl groups of the cellulose fibers *via* modification with glycidyl methacrylate, or alternatively on the carboxyls resulting by an oxidization step to the cellulose fibers such as TEMPO-oxidation *via* modification with an aminoalkyl methacrylate, such as 2-aminoethyl methacrylate. Therefore, the methacrylated cellulose nanofibrils may comprise cellulose nanofibrils methacrylated either on the OH groups or on the COOH groups present on anionic cellulose fibers.

In one embodiment, the methacrylated cellulose nanofibrils have a degree of substitution (DS) of methacrylates (methacrylate modification degree) in the modified nanocellulose. Preferably, the degree of substitution is from 0.005 to 0.5, more preferably 0.01 to 0.03, such as about 0.02.

### Crosslinking agents

In one preferred embodiment, the hydrogel formulation comprises a crosslinking agent. In a further preferred embodiment, the hydrogel formulation comprises at least one Photo-/UV-crosslinking agent (photoinitiator). Photo/UV crosslinking agents contain groups, which will give rise to cross-links for example under the influence of UV-radiation. The wavelength for the radiation used for achieving crosslinking of UV crosslinkers is generally in the range of 100 to 400 nm. In one embodiment, a wavelength of 312 nm (falling within the UVB range) is employed and in a second embodiment a wavelength of 254 nm (falling within the UVC range) is used.

The actual chemical identity of the crosslinker is not critical. However, preferably the crosslinking agents or photoinitiators are biocompatible. Typically, the photoinitiators are water-soluble and biocompatible or are used at non-cytotoxic concentrations. Further typically, in case of UV-crosslinking agents, they are compounds, which contain groups, which can be activated by UV radiation to achieve chemical or physical reactions. Such groups contain unsaturation, typically as double or triple bonds, for example carbon-carbon double bonds or carbon-nitrogen double or triple bonds, and optionally oxygen groups such as oxo or carboxy or epoxy groups. Acrylates, alkacrylates, carbodiimides, vinyl, allyl or glycidyl groups can be mentioned as examples. Crosslinking can also be achieved with alkene hydrothiolation.

In one embodiment, UV-crosslinking agents may be selected from the group of benzophenone, citric acid, and diamines and similar monomeric compounds. In one embodiment, UV crosslinking agent comprises monomeric acrylamide (AA), preferably together with another crosslinking agent or photoinitiator.

In one embodiment, polymeric crosslinking agents are used, optionally containing acrylate or alkacrylate groups, such as methacrylates. Such compounds are exemplified by polyvinyl alcohol, poly(methyl vinyl ether-co-maleic acid), 1, 4-butanediol diglycidyl ether and pentaerythritol triacrylate.

In one embodiment, the crosslinkers are UV-crosslinking agents comprising cross-linkable polymers, such as polysaccharides, e.g. obtained from wood, containing cross-linking groups, such as acrylate or alkacrylate groups, such as methacrylates. Such UV-crosslinking agents can be selected from, for example the group of methacrylate (MA) modified hemicelluloses, in particular wood-derived hemicelluloses, for example galactoglucomannan (GGM).

In one embodiment, the crosslinking agent is selected from methacrylated biopolymers, typically biopolymers selected from gelatin, chitosan, alginate and wood polysaccharides, such as galactoglucomannan, xylan and xyloglucan, and combinations thereof.

In one embodiment, the methacrylated biopolymer comprises gelatin methacrylate, methacrylated galactoglucomannan or a combination thereof.

In one embodiment, the hydrogel formulation comprises a photoinitiator and a crosslinking agent selected from methacrylated biopolymers, monomeric acrylamide, methacrylic anhydride and combinations thereof, in addition to methacrylated cellulose nanofibrils.

### Biological material

As stated above, in some embodiments the present ink formulation is used for preparing a bioink formulation. Such a formulation can be obtained by admixing a composition, in particular an aqueous composition, such as an aqueous suspension, of biological material with the ink formulation comprising methacrylated cellulose nanofibrils, thus obtaining a bioink formulation, which comprises an ink formulation together with biological material.

In an embodiment, the biological material is selected from the group of vegetable and animal cells and tissue, in particular from the group of endothelial cells, fibroblasts, stem cells, chondrocytes and osteoblasts.

In one embodiment, cells, such fibroblast or endothelial cells, are incorporated into an ink formulation of the present kind in the form of growth media containing such cells. The growth medium is typically formed by an aqueous phase containing in addition to cells also other components, such as inorganic and organic compounds in dissolved or dispersed (suspended) form.

The aqueous phase of the ink formulation or of the nanocellulose hydrogel may contain organic and inorganic components, such as salts and other residues stemming from the manufacturing of the nanocellulose component and the suspension of biological material.

In one embodiment, the aqueous phase of the cellulose nanofibril hydrogel contains non-solubilized monomeric, oligomeric and polymeric compounds derived from the production of the nanocellulose hydrogel from biomass or other feedstock. Typically, the content of such additional components does not exceed 5 %, in particular not 2.5 %, preferably not 1 %, by weight of the hydrogel. Such components are included into the ink composition when the nanocellulose hydrogel is combined with UV cross-linkers and biological matter.

The aqueous phase of the ink composition may further contain residues of amino acids, vitamins, inorganic salts, glucose, growth factors, and hormones. Such components are present in one embodiment, wherein the biological material mixed with the methacrylated nanocellulose hydrogel is provided in the form of a culture medium containing cells. Typically, the content of such additional components does not exceed 5 %, in particular not 2.5 %, preferably not 1 %, by weight of the total composition.

In one embodiment, the aqueous phase of the ink composition contains other components than methacrylated cellulose nanofibrils, UV crosslinking agent and biological material up to 10 %, in particular not more than 5 %, for example not more than 2.5 % by weight of the total composition.

### Method of producing the hydrogel formulation

The present ink formulations suitable for 3D printing can be manufactured by a method comprising the steps of
- providing an aqueous gel of methacrylated cellulose nanofibrils,
- optionally providing at least one crosslinking agent, preferably a photoinitiator
- optionally providing an aqueous dispersion of biological material, and
- mixing the gel, the optional crosslinking agent and optionally the aqueous dispersion of biological material to form an ink composition,
wherein the cellulose nanofibrils have a charge density of 1.2 mmol/g or lower.

In one embodiment, the present ink formulations suitable for 3D printing can be manufactured by a method comprising the steps of
- providing an aqueous gel of methacrylated cellulose nanofibrils,
- providing a water-soluble photoinitiator and optionally providing at least one additional crosslinking agent,
- optionally providing an aqueous dispersion of biological material, and
- mixing the gel, the photoinitiator and the optional additional crosslinking agent and optionally the aqueous dispersion of biological material to form an ink composition,
wherein the cellulose nanofibrils have a charge density of 1.2 mmol/g or lower.

In one embodiment, the aqueous gel of methacrylated cellulose nanofibrils is produced by subjecting cellulose nanofibrils to grafting methacryloyl groups to the fiber surface through reacting with either the hydroxyls or carboxyls within anionic cellulose fiber structure, wherein the grafting step comprises:
(a) modification with acrylic anhydrides, such as methacrylic anhydride; or
(b) modification with glycidyl methacrylate; or
(c) modification with 2-aminoalkyl methacrylate, such as 2-aminoethyl methacrylate.

In one embodiment, the aqueous gel of methacrylated cellulose nanofibrils is produced by
- oxidizing cellulose fibers in an aqueous medium,
- subjecting the oxidized cellulose to solvent exchange from water to an organic solvent, preferably to dimethylformamide,
- surface modification of hydroxyl groups on cellulose fibers with acrylic anhydrides, such as methacrylic anhydride or acrylic anhydride, preferably with methacrylic anhydride,
- removal of the organic solvent and mechanical defibrillation of the methacrylated cellulose fibers in water to obtain the aqueous gel of methacrylated cellulose nanofibrils.

In one embodiment, the ink formulation is produced by mixing a predetermined amount of a crosslinking agent with the aqueous gel of methacrylated cellulose nanofibrils containing 0.1 to 3% of cellulose nanofibrils to provide a modified hydrogel containing 0.1 to 3% of cellulose nanofibrils and about 0.1 to 5%, of a crosslinking agent, calculated from the total weight of the formulation. Typically, the rest of the formulation comprises water. The water content of the formulation may thus be at least 92 %, preferably at least 95 %, based on the total weight of the formulation.

A stated above, the cellulose nanofibrils have a charge density of 1.2 mmol/g or lower, preferably less than 1.2 mmol/g, in particular less than 1.0 mmol/g, suitably less than 0.9 mmol/g, such as less than 0.8 mmol/g, in particular the cellulose nanofibrils are free of charge or essentially free of charge.

In one embodiment, the crosslinking agent is a Photo-/UV-crosslinking agent selected from monomeric acrylamide or methacrylated biopolymers, optionally together with another Photo/UV-crosslinking agent or photoinitiator. In one embodiment, the crosslinking agent is preferably added in the form of a powder or an aqueous suspension to the aqueous gel of methacrylated cellulose nanofibers.

In a further embodiment, biological material may be included in the hydrogel formulation. Thus the method of producing an ink formulation for 3D printing may comprise the steps of
- providing an aqueous suspension of biological material selected from the group of vegetable and animal cells and tissue, in particular from the group of endothelial cells, fibroblast, stem, chondrocyte and osteoblast,
- mixing the suspension with the aqueous hydrogel of methacrylated cellulose nanofibers, and
- adding a Photo-/UV-crosslinking agent at a preselected step.

In one embodiment, the method comprises the steps of providing an aqueous gel of methacrylated cellulose nanofibrils, providing a photoinitiator, providing a monomeric acrylamide or methacrylated biopolymer, such as methacrylated galactoglucomannan, or both, optionally providing an aqueous dispersion of biological material, and mixing the gel, the photoinitiator, the crosslinking agent and optionally the aqueous dispersion of biological material to form an ink composition.

In one embodiment, the ink formulation is produced by mixing a predetermined amount of a photoinitiator and acrylamide and/or methacrylated biopolymer with the aqueous gel of methacrylated cellulose nanofibrils containing 0.1 to 3% of cellulose nanofibrils to provide a modified hydrogel containing 0.1 to 3% of cellulose nanofibrils, about 0.1 to 5%, of a photoinitiator and about 0.1 to 4% of acrylamide and/or methacrylated biopolymer, calculated from the total weight of the formulation. Typically, the rest of the formulation comprises water.

By using non-charged or lowly charge nanocellulose of the above kind in the ink formulation, preferably together or in combination with cross-linking agents, bioinks can be formulated where cells and cell media can be added without the addition giving cause to phase separation prior to 3D printing.

A phase-instable ink typically exhibits aggregation of polymeric species and phase separation. Such instability can be assessed visually, for example using an optical microscope. However, typical light-scattering devices, e.g. zeta-sizer (Malvern), can also be applied.

The result of phase-instability is that the ink cannot be printed by the defined capillary channel or needles or in a homogenous form. It often means that if aggregates are formed, the size of those will be bigger than the diameter of the capillary channel or needles.

In one embodiment, the bioink formulation is at least essentially stable upon standing at least 12 hours, typically 16 to 120 hours, for example at least 24 hours at room temperature (20 °C ± 5 °C). This means that there is no or essentially no aggregation of polymeric species or other species present in the composition. In one aspect, the stability of the bioink means that the composition can be used for 3D printing over a time period of at least 12 hours, typically 16 to 120 hours, for example at least 24 hours, from the point of time when the ink composition and the composition of biological matter were mixed together to provide a bioink composition.

By contrast, compositions according to the present technology and embodiments described herein can be printed for example using extrusion-based 3D printing wherein typically micro-syringes are used having a capillary with a diameter, in transversal direction, of 100 to 500 µm, for example about 100 to 300 µm or about 150 to 200 µm.

In one embodiment, the bioink composition is essentially free from aggregates having a size greater than 1000 µm, in particular greater than 600 µm, for example greater than 500 µm, observed by TEM imaging. As a result, the pressure drop in the micro-syringe of the 3D printer using the bioink will typically be less than 50 %, in particular less than 30 %, for example less than 10 % compared to the corresponding hydrogel-UV crosslinker composition containing no biological matter.

Using a 3D ink formulation of the above kind, scaffolds can be produced having a compressive Young's modulus in the range of 1 to 50 kPa, in particular about 2 to 40 kPa.

It has been found that by virtue of the lack or very low extent of cell separation of the bioink, scaffolds can be produced having a combination of good mechanical properties and an even distribution of the biological material throughout the hydrogel. It has further been found that by improving the mechanical properties of the scaffold, increased proliferation of cells can be reached. In particular, there is a positive correlation between the mechanical stiffness of the hydrogel scaffold and the proliferation of cells in the scaffold.

It is to be understood that terminology employed herein is used for the purpose of describing particular embodiments only and is not intended to be limiting.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

### EXPERIMENTAL

**Preparation of oxidized CNF with TEMPO:** TEMPO oxidized CNF was prepared from Spruce Kraft Pulp (produced by Borregaard, Norway) by the method described by Iotti et al, 2011. The oxidized fibres with charge density of 1.25±0.03 mmol/g were used for subsequent modification.

**Preparation of CNF-MA:** 128 g of the 7.8% TEMPO oxidized fibre in water (1.25 mmol/g charge, 10 g dry mass) was pressed on filter to remove water. Then the fibre mat was dispersed in 300 ml of DMF and filtered to remove most of the solvents followed by washing with 150 ml of DMF three times. The washed oxidized fibres were redispersed in 300 ml of DMF, to which, 10 ml (73 mmol) of trimethylamine was added. Afterwards, 8.5 ml (56 mmol) of methacrylic anhydride was added dropwise during 15 min. The reaction mixture was left stirring overnight with light protection. The thus-modified fibre was filtered and washed with water (300 ml, 5 times) and then concentrated to approximately 10% of the dry weight. The fibre dispersion in water was stored in cold with light protection until homogenization prior to further use.

### Preparation of hydrogel formulations

For example the following formulations were tested:
1. 1.1 % CNF-MA, 0.2 % Irgacure 2959
2. 1.1 % n/m-CNF, 1 % acrylamide, 0.4 % Irgacure 2959
3. 1.1 % CNF-MA, 1 % acrylamide, 0.4 % Irgacure 2959

Several other formulations containing varying concentrations of a crosslinking agent (for example acrylamide at a concentration of 0 to 4%), photoinitiator (0 to 0.4%, for example Irgacure 2959), and CNF-MA (for example from 0.85 to 1.3%) were also evaluated.

### Characterization

Charge density. The charge was determined for the prepared oxidized fibre by conductometric titration by the protocol described by da Silva Perez et al, 2003. CNF-MA charge was found to be 1.07±0.02 mmol/g.

Degree of substitution. The prepared fibre was analysed by NMR spectroscopy. NMR spectra were recorded with Bruker Avance 400MHz and 500MHz NMR spectrometers using standard pulse sequences. After sodium ions removal the CNF-MA was dissolved in the electrolyte containing 20% of n-Bu₄P⁺OAc⁻ and 80% of DMSO-d6. The solution was characterized by diffusion filtered 1H-NMR using methacrylic group signals compared to C1-H signal of glucose or glucouronic acid unit of cellulose for quantification of the DS of modification. It was determined the amount of the methacrylic groups of 2.0 mol% of the glucose or glucouronic acid unit of cellulose material which corresponds to DS 0.02 or ≈0.12 mmol/g.

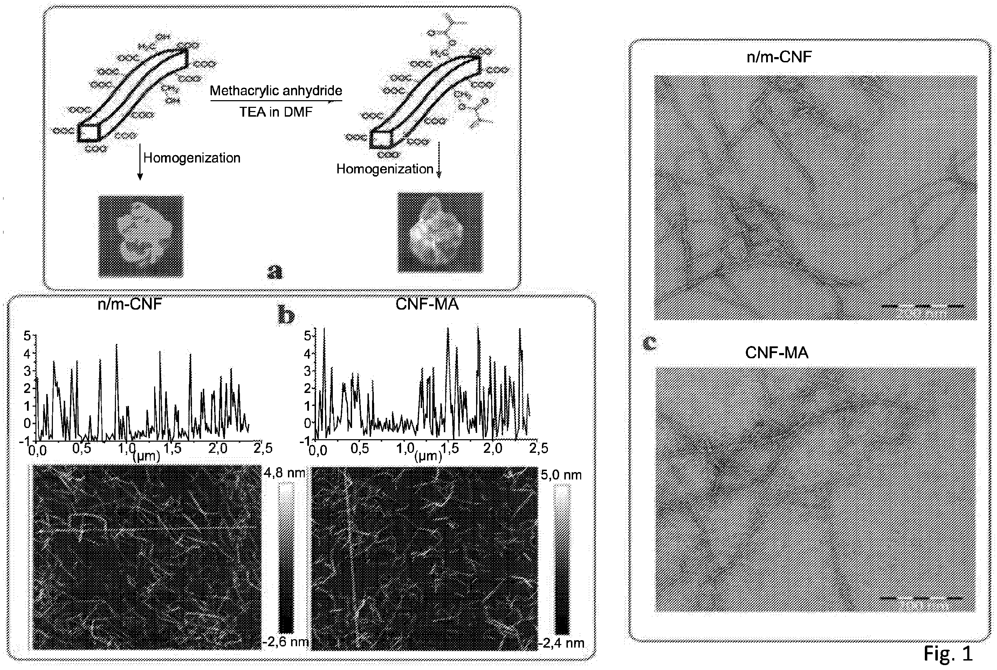

AFM imaging was performed for the visualization of the individual fibrils of CNF-MA and n/m-CNF. For the imaging Bruker MultiMode 8 with NCHV-A probe was utilized. Imaging was performed in tapping mode with tip radius around 10 nm was applied. The surfaces were prepared on clean silicon wafers. The CNFs were adsorbed by first adsorbing PEI (0.1 g/L) followed by correspondent CNF at the concentration of 0.1 g/l. The images with most representative height section are presented at Figure 1b.

TEM images. Transmission electron microscopy images were acquired by Jeol Transmission Electron Microscope. TEM images were captured for diluted solutions (0.01 g/l) of CNF-MA and n/m-CNF. Staining was performed by 0.1% uranyl acetate solution. Most represented images for the materials are presented on Figure 1c. Thickness of the fibrils and fibril length were determined from the images. Value of 3-5 nm for thickness and 300-1600 nm for length of the fibrils are in a good agreement with AFM and DLS results.

Supercritical CO₂ drying was performed by the instrument Autosamdri-815 with 20 min liquid CO₂ purging time.

BET surface area analysis was made using Micromeritics 3flex instrument using VacPrep061 degasser prior the nitrogen adsorption measurement. 2 aerogels samples for the BET nitrogen sorption analysis were prepared from 1.1 wt% CNF-MA and 1.1 wt% n/m-CNF hydrogel materials cross-linked by CaCl₂ solution. Water it the samples was gradually changed to ethanol followed by carbon dioxide critical point drying to preserve the original structure of CNF. The samples were degassed for 12 h at 60°C before the BET measurement. The analysis resulted in surface area of: 456 m²/g for CNF-MA aerogel and 441 m²/g for n/m-CNF material. Pore volume was 0.20 cm³/g and 0.19 cm³/g for CNF-MA and n/m-CNF materials respectively.

Photorheology. Rheology studies were carried out using TA Discovery HR-2 Rheometer with Peltier Plate and UV LED Curing Pate and 20 mm and 25 mm flat geometries. Rheological experiment was used to evaluate changes in mechanical properties which take place in the hydrogel under UV curing. Time sweep oscillation 5 min long with constant strain of 1% and shear rate of 1 s⁻¹ was used. UV (power varied from 15 to 32.5 mW/cm²) was switched on after 50 seconds of oscillation.

With this method (UV power 15 mW/cm²) were evaluated first 3 formulations consisted of:
4. 1.1 % CNF-MA, 0.2 % Irgacure 2959
5. 1.1 % n/m-CNF, 1 % acrylamide, 0.4 % Irgacure 2959
6. 1.1 % CNF-MA, 1 % acrylamide, 0.4 % Irgacure 2959

The results are presented in Figure 2a. To optimize the amount of the initiator Irgacure 2959, the tests (UV power 15 mW/cm²) presented at Figure 2b were performed. Content of the tested gels was: CNF-MA 1.1%, acrylamide 1%, Irgacure 2959 0-0.4%. The variations of the amount of acrylamide presented at Figure 2c. Content of the tasted materials was: CNF-MA 1.1%, acrylamide 0.25-4%, Irgacure 2959 0.2%, UV power 15 mW/cm².

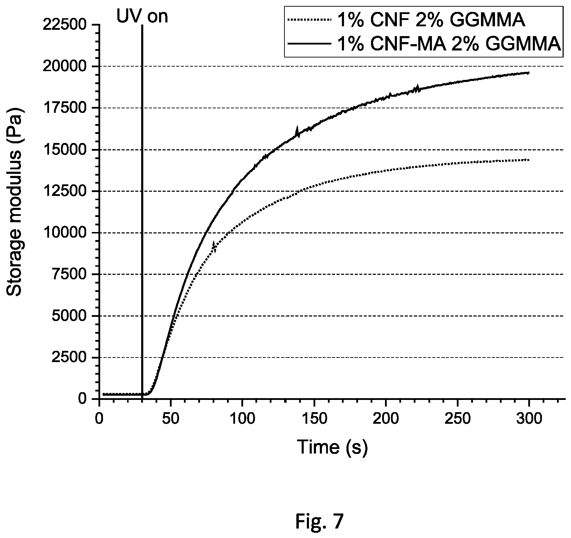

Further, a comparison between the mechanical properties of CNF and CNF-MA was carried out using photorheology studies. The results of CNF-GGMMA and CNF-MA - GGMMA are presented in Figure 7.

**Compression** tests. Compression tests were performed on TA Discovery HR-2 Rheometer with Peltier Plate and 25 mm flat geometry. For the additional characterisation of the properties of the materials compression tests were performed. Samples for the tests were prepared from the formulation containing 1.1% CNF, 1% acrylamide and 0.2% Initiator Irgacure 2959 (Ink-1).

Cylindrical mold (9.6 mm inner diameter, 50 mm length) was filled with the formulation. The filled mold was crosslinked by UV irradiation (Cellink Inkredible 3D printer integrated UV LED 365nm, 2 cm from the source) for 5 min. Prepared long cylinder with the diameter 9.6 mm was cutted by a razor to small cylinders with height 6-8 mm.

3 samples were airdried for 24h (21⁰C, 25% relative humidity) and reswelled for 2h (the same diameter formed). Few samples were soaked in PBS for 24 h (shrinked to diameter 8.9 mm). For the reference, the Ink-1 formulation (10 ml) was cross-linked with by addition of 0.1 ml of 5% CaCl₂ solution in the mold (after 48 h it was cutted to similar samples).

The compression test was performed using rheometer with compression speed of 500 µm/min. The compression force profiles were converted to pressure profiles using appropriate sample surface areas (P=F/a; a=πd²/4, d=9.6 mm for all the samples besides the PBS soaked where d=8.9 mm).

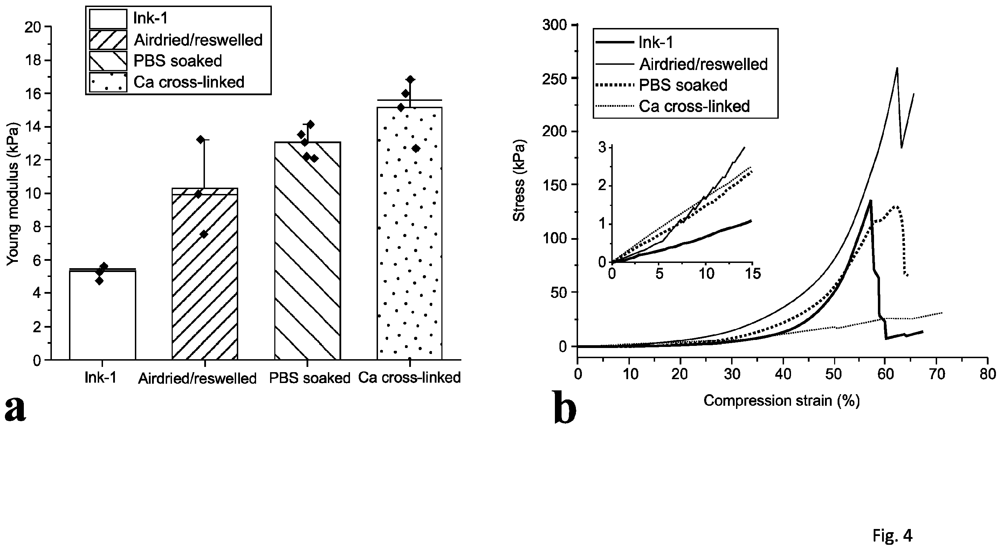

Young Modulus was determined as the slope of linear approximation of compression curve in the initial linear part (0-10% compression for all the samples besides 0-4% for airdried/reswelled samples) (E=P/σ). The results are presented on Figure 4a.

More samples were prepared for the compression analyses using the same procedure. Materials containing CNF-MA 1.1%, Irgacure 2959 0.2% and acrylamide with the concentration 0.25, 0.5, 1, 1.5, 2 and 4% were used for the crosslinking in the cylindrical molds. After cutting to small cylinders the samples were tested as described above. The most representative compression curves for the tests are presented at Figures 2e and 4b.

Young modulus values were calculated for the samples with different acrylamide concentrations. The dependence of the Young's modulus on the logarithm of the concentration of the acrylamide was linearly approximated with good correlation (Figure 2f).

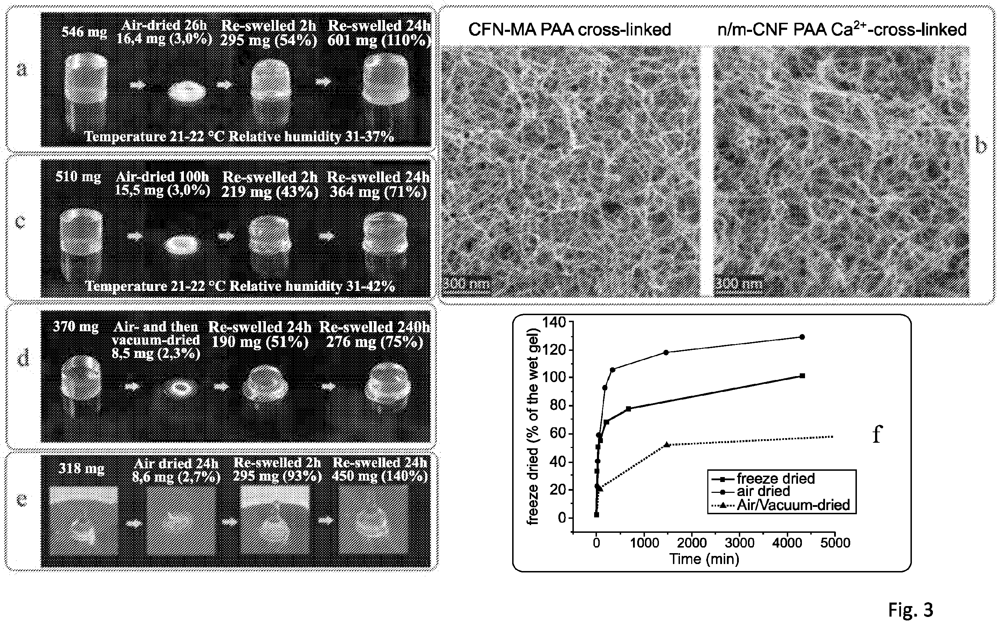

Morphology of the surfaces was characterized by SEM imaging using Leo 1530 Gemini instrument. The aerogels materials for the SEM imaging were prepared from UV-cross-linked "Ink-1" formulation by gradual changing the solvent from water to ethanol followed by carbon dioxide critical point drying. The procedure of the aerogel preparation was performed to preserve the original structure of the hydrogel. For the comparison sample was prepared from the formulation of 1.1% n/m-CNF, 1% acrylamide and 0.2% of Irgacure 2959. Acrylamide in the sample gel was polymerized by UV and then the gel was cross-linked by addition of CaCl₂ 5% solution. The material was treated the same way as the first sample to get the aerogel. The aerogels were sputter-coated with platinum prior imaging. The SEM images of the cross-section surfaces of the materials presented on the Figure 3b.

Swelling behaviour. To investigate the prepared material ("Ink 1" - 1.1% CNF, 1% acrylamide and 0.2% Initiator Irgacure 2959, cross-linked with UV) for the swelling ability 2 sets of samples were prepared. Cross-linked gel cylinders were prepared as described for the compression tests. 4 samples were left for 24h for air-drying at room temperature conditions (22 ⁰C, 25% relative humidity). Weight of the residue was 2.5% (average of 4 samples) of the original gel (dry content 2.3 %, the difference of ~10% could be residual moisture content.

3 samples were freeze-dried for 18h to give 2.48% (average of 3 samples) of the original gel (dry content 2.3 %, the difference of ~8% could again be residual moisture and an error of the concentration in the prepared gel. The samples were placed to the deionized water and kinetics of water uptake was determined. The recorded data for the air-dried samples (average of 4) and for freeze-dried samples (average of 3) are presented at Figure 3f.

To investigate how the time of drying effect the swelling behaviour 2 samples were prepared from the Ink 1 formulation and crosslinked by UV curing. The samples were airdried for 24h and 120h (20⁰C, 25% relative humidity) to give the shapeless residues of 2.7% and 2.5% of the original gels respectively. The samples were placed in water for swelling. After few hours the first sample restored the original shape completely (Figure 3a). The other sample did not fully restore the shape after few days of swelling (Figure 3c).

To investigate the effect of the water residue to the swelling ability 2 samples of the cross-linked Ink-1 material was air-dried for 24h and then vacuum-dried at high vacuum and room temperature for 20h. The result of swelling in water for this material presented at the Figure 3e. Kinetic of swelling of the material compared to other conditions is presented at Figure 3f.

### Cell assays

Cell culture. Human dermal fibroblasts (HDFs) and Hela cells were used for the biocompatibility evaluation of CNF-MA hydrogels. Both cell lines were maintained in 10-cm petri dishes in a humid CO₂ incubator with preset temperature at 37°C. Cell medium was changed every two days with Dulbecco's Modified Eagle high glucose Medium (DMEM, high glucose) supplemented with 2 mML-glutamine, 100 IU/mL penicillin/ streptomycin(P/S) and 10% heat-inactivated fetal bovine serum (FBS). Cells were passaged upon reaching 70% percent confluency.

Hydrogel coating. CNF-MA (1.1%) hydrogels containing 0.25%, 0.5% and 1% AA were dispensed in 96-well plates and 24-well plates, respectively. Coated plates were washed with sterile Dulbecco's phosphate-buffered saline (DPBS) 3-5 times to remove potential free acrylamide monomers and were stored in +4°C fridge until further experiments. Hydrogel-coated plates were pre-incubated with supplemented DMEM overnight in humidified CO₂ incubator before plating cells.

Cell proliferation assay and cytotoxicity assay. 100 µL of HDFs and Hela cell suspensions were dispensed respectively in 96-well plates (5000 cells/well) pre-coated with different CNF-MA hydrogels as mentioned above. Seeded plates were incubated in the CO₂ incubator at 37 °C for 1, 2, 3, 5, 7 days. Cell culture medium was changed regularly every two days. At the end of each time point, 10 µL of CCK-8 solution (Donjito) were added to each testing well of the plates. Absorbance was measured at 450 nm using a microplate reader after 1 hour incubation at 37°C.

Immunofluorescent staining and confocal microscopy. HDF and Hela cells were seeded in 24-well glass-bottom plates pre-coated with different CNF-MA hydrogels as mentioned above. Cells were fixed with 4% paraformaldehyde for 15 min and stained with phalloidin (Ex.647 nm) and DAPI (Ex.405 nm) subsequently. Cells were mounted with PBS with 0.03% NaN3 in plates. Z-stack Images were taken using confocal microscopy (3i CSU-W1 spinning disk, 10x Zeiss Plan-Apochromat, NA=0.8). Images were processed with ImageJ where maximal projection was applied to show maximal intensity.

### Results

### Methacrylated cellulose nanofibers (CNF-MA): Synthesis, surface charge vs DS of methacrylates, fibril morphology

Methacrylated nanocellulose (CNF-MA) was synthesized to enable *in-situ* polymerization of MA moiety anchored on the fibril surface. TEMPO-mediated oxidation was applied to activate the fiber surface prior to methacrylation and resulted in cellulose fibers with charge density of 0.91, 1.25, and 1.40 mmol/g, respectively (Table 1). Afterwards, water was exchanged to DMF in order to increase the reaction efficiency of further derivatization with methacrylic anhydride. The oxidized fiber with charge density of 0.91 mmol/g achieved highest DS for MA, 0.03. Higher charge density had a negative impact on the DS for MA. That can be explained by the less availability of hydroxyl groups in the oxidized fiber with higher content of carboxylate. It is also observed that the amount of the carboxylate groups after MA derivatization has decreased, which is most probably ascribed to hydrolysis that might have occurred during solvent exchange from water to DMF and back to water. The oxidized and methacrylated fibers were homogenized in water suspension to produce nanofibrillated fibers (n/m-CNF and CNF-MA, respectively).

**Table 1. Modification degree of MA-group vs fibre charge before and after modification**

| | Fibre charge, mmol/g (DS of oxidation) | | |
|---|---|---|---|
| | before modification | after modification | DS of MA-group |
| I | 1.40±0.07 (0.231) | 1.18±0.05 (0.194) | 0.014 |
| II | 1.25±0.03 (0.206) | 1.07±0.02 (0.175) | 0.02 |
| III | 0.91±0.04 (0.149) | 0.82±0.03 (0.134) | 0.03 |

Modification of the cellulose fibre surface especially in organic solvent often alters the structural properties of fibres. Thus, the modified fibrils were thoroughly characterized to assess their morphological and chemical properties. AFM imaging of the individual fibrils of the modified and non-modified CNF (Figure 1b) showed similar dimensions, with a width in the range from 3 to 5 nm for both samples. The same fibre width range is also observed by TEM imaging (Figure 1c). The fibre length is shown in the range from 200 nm to a couple of micrometers. Furthermore, surface areas of the aerogels prepared from both n/m-CNF and CNF-MA hydrogels with supercritical CO₂ drying were measured by BET nitrogen adsorption method. Both materials showed close surface area (420 m²/g for non-modified and 450 m²/g for the CNF-MA) and similar pore size distribution.

### UV crosslinkable hydrogels resulted by copolymerisation of CNF-MA and acryl amide (AA): photochemistry kinetics, mechanical characteristics and swelling behaviour of hydrogels of CNF-MA+AA

The CNF-MA was copolymerized together with the presence of acrylamide monomer under the UV-irradiation at 365 nm. Here, acrylamide was chosen because of the accessibility and good bio-mechanical properties of the polyacrylamide in water. CNF-MA with 1 mmol/g surface charge and 0.02 methacrylate modification degree was selected, as an adequate level of surface ionization is essential to achieve more homogenized defibrillation of cellulose nanofibers in the final product, but an intermediate level of surface charge of -COO⁻ has been proven to better support the proliferative activity of cell lines, such as fibroblasts (Kummala et al, 2020; Liu et al, 2016). To reveal the photochemistry kinetics, the storage modulus of G' upon UV irradiation was registered in an oscillatory mode with a constant strain of 1% and frequency of 1 s⁻¹ as shown in Figure 2a. Irgacure 2959 photoinitiator concentration initially set at 0.4%. G' of CNF-MA (1.1%) showed a very weak enhancement upon the UV exposure, but no hydrogel with an affordable integrity was resulted for easy handling. This suggests that the crosslinking of methacrylate occurs in the pristine CNF-MA but not sufficient to support a self-sustained network. As expected, the addition of 1% acrylamide into 1.1 % CNF-MA showed a rapid and profound G' increase upon UV-irradiation, which in turn gave an elastic hydrogel with good integrity as shown in Figure 2d. As a control to compare, the G' of 1 % n/m-CNF+1 % acrylamide formulation remained unchanged under UV exposure, which proved that the copolymerization of CNF-MA and acrylamide was accounted for the formation of interpenetrating polymeric network. While not wishing to be bound the theory, it is believed that the polyacrylamide acts as spacer to create the fibril-fibril linkages in the hydrogel.

In order to optimize the photoinitiator concentration, the photochemistry kinetics of 1.1 % CNF-MA+1 % acrylamide formulation was registered with various concentrations of Irgacure 2959 from 0 to 0.4 %. The responses of G' upon UV-irradiation are displayed in Figure 2b. In general, a higher photo initiator concentration favoured a faster UV-curing. When the concentration of Irgacure 2959 was above 0.1%, a completed degree of crosslinking (G' reaching a plateau) can be achieved within a UV exposure duration less than 30 seconds, as desired in the UV-aided DIW printing to support good ink fidelity. 0.2 % Irgacure 2959 was selected as optimal for further use, as too high concentration of Irgacure 2959 might have a risk to cause cell toxicity.

Optimization of the acrylamide crosslinker content via the photorheology tool was also performed with the CNF-MA content set as 1.1% but the acrylamide content varying from 0.25 to 4%. The responses of their G' upon UV-exposure are presented in Figure 2c. In the content range as studied, although the impact of the acrylamide content on the photochemistry kinetics was minor, a faster curing was supported when the acrylamide content was higher that 1.0 % than in the cases of 0.25 and 0.5% (as determined by the duration taken to reach 90% of the plateaued value of G'). Meanwhile, a larger content of acrylamide resulted in a higher value of G' for the UV-crosslinked hydrogel of CNF-MA+PAA, which indicates a stronger viscoelastic properties as increasing the content of monomeric crosslinker. Still, the UV-crosslinked hydrogels of CNF-MA+PAA was shown to be relatively soft materials with the G' values less than 1.5 KPa. Meanwhile, the mechanical response of this group of UV-crosslinked CNF-MA+PAA hydrogels was measured by the axial compression test, as depicted by the stress-strain curves in Figure 2e. The CNF-MA+PAA hydrogels possessed remarkable mechanical properties good elasticity with a fracture strain > 55% when the monomeric acrylamide content was above 1.0%. In Figure 2f, the Young modulus derived from the stress-strain curve was displayed for all four repetitive samples in each catalogue of the acrylamide content at 0.25, 0.5, 1.0, 2.0, or 4.0%. The Young modulus showed a linear dependence on the logarithm of acrylamide content in the formulation, as a higher crosslinking density creates a stiffer network. As the toughest gel as prepared, the UV-crosslinked 1.1% CNF-MA+4% PAA hydrogel can reach 0.45 MPa for the compressive stress at a compressive strain of 68%.

In targeted applications such as biomaterial matrix in supporting 3D cell culture, to dry the CNF-based hydrogels into films/aerogels and to reswell it in the applicable medium would be a convenient approach for storage and transportation of the hydrogels point of view in practical scenarios. With the hydrogels prepared by UV cross-linking of 1.1% CNF-MA+1% acrylamide formulation, we further investigated the reswelling behaviours after various drying conditions. As illustrated in Figure 3a, a cylinder shaped hydrogel was air-dried for 24 h to get a dry film; the film reswelled for 24h in deionized water and ideally restored its original geometry. Under morphological analysis by SEM in Figure 3b, the aerogel of 1.1% CNF-MA+1% PAA that was prepared by supercritical CO₂ drying showed a well-developed and 3D connected network of cellulose fibrils. Furthermore, no distinct variation was seen in comparison with n/m-CNF aerogel cross-linked by Ca²⁺, as a control sample. The highly porous but interconnected fibril network is accounted for the excellent reswelling capability of the dried film. More detailed investigation revealed that extended air-drying time (100 h) and vacuum-drying would decrease water-uptake speed in reswelling and deteriorate its capability to restore its original geometry, as seen from the samples displayed in Figure 3c and 3d. Under these drying conditions, the fiber hornification led to lower porosity and poorer solvent accessibility and deteriorated the reswelling capability of the dry films. In addition, the excellent shape-memory effect of the 1.1% CNF-MA+1% PAA hydrogel (upon 24 h air-drying) was also demonstrated with a hydrogel 'teat' in even more complex geometry, as seen in Figure 3e.

With the 1.1% CNF-MA+1% PAA hydrogel under investigation, the water uptake (reswelling) speed was carefully monitored for dried samples prepared from three different drying methods: air-drying for 24 h, air-drying 24 h followed by vacuum-drying and freeze-drying. As displayed in Figure 3f, both air-drying sample and freeze-drying sample have higher speeds of water uptake than the vacuum-dried sample. When reaching the swelling equilibrium, the air-dried sample swelled in 3 h back to the mass of the original hydrogel and swelled to almost 130% in 24 h; the freeze-dried sample restored the mass of original hydrogel only after 24 h of swelling. However, the vacuum-dried sample could not swell back to its original mass with reaching only 50% water uptake after 24 h of immersion.

Considering to apply the as-prepared CNF-MA+PAA hydrogel in supporting 3D cell culture, the integral stability of the 1.1% CNF-MA+1% PAA hydrogel was evaluated in the PBS buffer. After being soaked in PBS buffer for 24 h, shrinking of the hydrogel sample was observed (around 10% of the linear dimensions). At the same time, the Young's modulus value for 1.1% CNF-MA+1% PAA hydrogel before (5.3 kPa) raised more than twice after PBS soaking (13 kPa) , as compared in Figure 4a. This is mainly due to the screening of the negative surface charge by the cations, which leads to closer fibril-fibril interactions in the hydrogel as the hydrogen bond interaction is prominent. Furthermore, the swollen hydrogel samples prepared by reswelling the air-dried 1.1% CNF-MA+1% PAA films in deionized water gave almost 2-fold increase in the Young's modulus as a reported value close to 10.3 kPa. It is indicated that the evaporation-swelling method greatly changes the internal network of the hydrogel and increases the network's crosslinking density, very possible by restructuring the hydrogel bonds among the cellulose nanofibrils. 1.1% CNF-MA crosslinked by 0.05 % Calcium chloride was used as a benchmark to compare the new materials with. It was observed that even though the 1.1% CNF-MA+1% PAA hydrogel (Young's modulus 5.3 kPa) is significantly softer than calcium-crosslinked (Young's modulus 15.2 kPa), the stress at break is higher for the polyacrylamide-cross-linked material for more than 3.5 fold (98.7 kPa for PAA-crosslinked vs 27 kPa for Ca-crosslinked). It is worth to notice no significant difference in stress at break for the PAA-crosslinked material before (98.7 kPa) and after (110 kPa) soaking in PBS and raising it 2 fold (to 215 kPa) for the airdried/reswelled material.

### Cytotoxicity and proliferation of fibroblasts and cancer cells on the CN-MA+PAA hydrogel

The cyto-compatibility of the cross-linked CNF-MA+PAA hydrogels as the cell culture matrix was preliminary evaluated in the cell culture of Human Dermal Fibroblasts (HDF) and HeLa cell lines. With the cell culture plastic as control, three types of CNF-MA+PAA hydrogels (cross-linked from 1.1% CNF-MA and acrylamide of varied concentration of 0.25%, 0.5%, and 1%) were compared in terms of the cytotoxicity and the cell proliferation behaviours. In the culture of HDF and HeLa cells, all three groups of CNF-MA+PAA hydrogels demonstrated satisfactory cell viability compared to the culture on two-dimensional (2D)-Mock. The confocal imaging analysis of the fixed matrices after 48 h incubation also suggest that all the hydrogel matrices supported both types of cell to grown on matrix surface, as indicated by the intense distribution of cells positive for two markers of actin cytoskeleton protein marker Phalloidin and the nuclear marker 4', 6-diamidino-2-phenylindole (DAPI). The elongated morphology of both HDF cells and Hela cells further demonstrates the good cyto-compatibility of both investigated cell lines growing on the hydrogel matrices.

Moreover, the CNF-MA+PAA hydrogels supported the cell proliferation after longer incubation periods. The cell proliferation was quantitatively evaluated at time points of day 1(D1), D2, D3, D5 and D7 as shown in Figure 5B iv for HDF and in Figure 5B iii for HeLa cells. At D1 and D2, the cell growth was slightly inhibited in comparison to 2D-Mock. After 72h, both HDF cells and HeLa Cells recovered high proliferation speed and the proliferation rate showed no significant deviation from the 2D-Mock control. Meanwhile, the cell proliferation made no distinguished variation among the three groups of CNF-MA+PAA hydrogels, which indicates that the content of polyacrylamide resulted in the hydrogels plays a minor role in affecting the gel's compatibility in supporting the cell proliferation or survival. In cell laboratories focused on mechanotransductive cell behaviours, the PAA-based hydrogels with a predesignated stiffness gradient have been widely used as *in vitro* cell culture platforms to study cellular behaviour in response to ECM elasticity. As the PAA hydrogels are lack of cell adhesive domains, it additionally requires a 'gel-activating' step with functionalization of Sulfo-SANPAH (sulfosuccinimidyl 6-(4'-azido-2'-nitrophenylamino)hexanoate) to subsequently covalently attach the ECM protein, such as fibronectin, laminin, or collagen. Featured with low dosage of monomeric AA in resulting sufficient crosslinking, ease of fabrication, and satisfactory cyto-compatibility, the CNF-MA+PAA hydrogels have a great potential as a novel matrix system in creating stiffness gradient hydrogels with a patterned or moving photomasks for UV photopolymerization.

### Validation of formulation of CNF-MA and AA as feedstock ink in DIW printing

3D printability and shape fidelity of the developed formulations were evaluated by extrusion-based 3D printer with a pneumatic dispensing system and conical polyethylene nozzle. The printability was optimized for nozzle diameter of 250 µm and printing speed of 600 mm/min. After the extrusion the scaffolds were cross-linked by the printer's integrated UV source. The formulations containing fixed amount of acrylamide (1%) and initiator Irgacure 2959 (0.2%) with CNF-MA concentration variations from 0.85 to 1.3% were evaluated for the printability. As the printing approach allows the cross-linking only after extrusion, the printed object is expected to keep its shape until the UV cross-linking is applied.

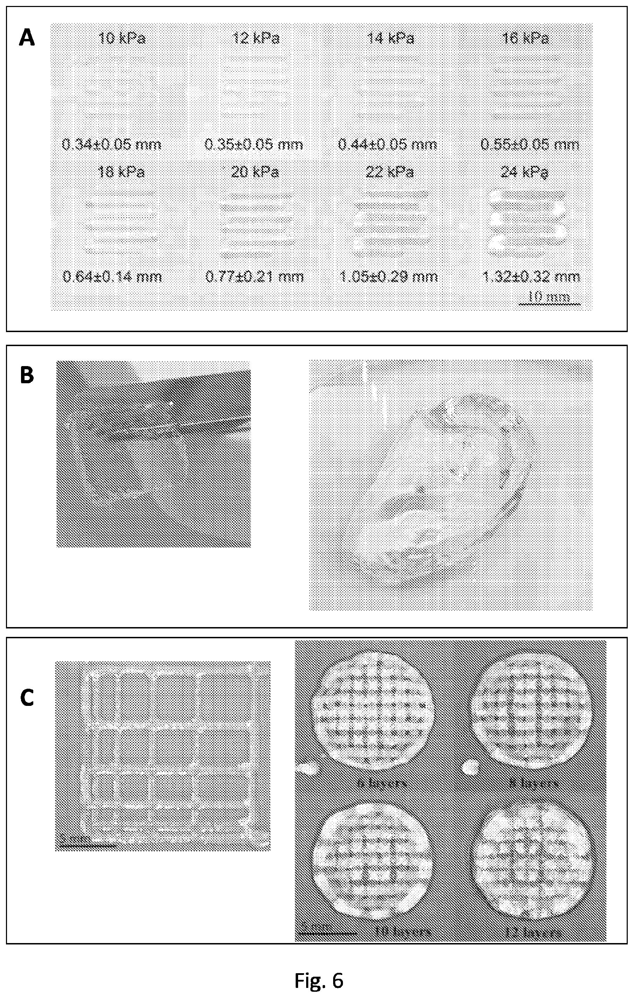

Formulation "Ink-1" with 1.1% of CNF-MA was determined to suit best the printing conditions. Optimal strut diameter (0.45 mm) for the "Ink-1" formulation was determined to extrude at 14 kPa pressure (Figure 6A). It was also shown that the material is suitable for printing the complex shape objects (Figure 6B). To further assess the resolution capacity of the ink formulation, UV cross-linking was applied during extrusion. The printed struts were clearly resolved at distance over 1 mm when 250 µm cylindrical stainless steel nozzle, 240 mm/min speed and 100% dispensing rate were set to print the calibration grids with 1-5 mm mesh (Figure 6C). In order to determine maximum number of layers deposited on each other without fusing, circular grid of 1.5 mm mesh with different amount of layers was also printed (Figure 6C). It was observed that up to 10 layers could be printed on top of each other without significant fusing of the grid. In compared to the previous studies where CNF hydrogel and such auxiliary methacrylate components as gelatine and biopolymer methacrylates were formulated, the current CNF-MA ink can only be used in manufacturing relatively soft hydrogel matrix applications. Thus, further development should be explored to extend the stiffness range of hydrogel by varying the crosslinker and increasing the DS of MA, which is proven to be a challenge while aiming at a relatively higher content of biopolymers in the formulation.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

### INDUSTRIAL APPLICABILITY

The ink comprising methacrylated cellulose nanofibers, preferably with crosslinking agent, can be formulated together with cells into bioinks that can be printed and used for cell-matrix and cell-cell interaction studies. Further, the ink comprising methacrylated cellulose nanofibers, preferably with a crosslinking agent, can be formulated together with cells into bioinks that can be printed and used for drug screening or drug therapy studies. The bioink can be used to produce 3D hydrogel scaffolds suitable as cell culture platforms. The scaffolds can be used for proliferating different cells, e.g. cancer cell. The scaffolds typically have a mechanical strength, which enables their use for example in cell-matrix and cell-cell interaction studies. They can also be used as cell culture platforms for drug screening or drug therapy studies. They can further be used as scaffolds for tissue and organ engineering and regenerative medicine.

### ACRONYMS LIST

- AA: acrylamide
- AFM: atomic force microscopy
- CNF-MA: methacrylated cellulose nanofibrils
- DIW: direct ink writing
- DMF: dimethylformamide
- DS: degree of substitution
- GelMA: gelatin methacrylate
- GGM: galactoglucomannan
- GGMMA: methacrylated galactoglucomannan
- PAA: polyacrylamide
- SEM: scanning electron microscope
- TEM: transmission electron microscope
- TEMPO: 2,2,6,6-tetramethylpiperidine-1-oxyl radical

### CITATION LIST

### Patent Literature

CN 110787320 B
US 2021171773 A1
Non Patent Literature
Da Silva Perez, D., S. Montanari, M. R. Vignon, Biomacromolecules 2003, 4, 1417
Iotti, M., Ø. W. Gregersen, S. Moe, M. Lenes, J. Polym. Environ. 2011, 19, 137
Kummala, R., Soto Véliz, D., Fang, Z., Xu, W., Abitbol, T., Xu, C. & Toivakka, M., 9 Mar 2020, Human dermal fibroblast viability and adhesion on cellulose nanomaterial coatings: Influence of surface characteristics. In: Biomacromolecules. 21, 4, p. 1560-156
Liu,J., Chinga-Carrasco, G., Cheng, F., Xu, C., Willför, S., Syverud, K. & Xu, W. Y., (2016) Hemicellulose-reinforced nanocellulose hydrogels for wound healing application. In: Cellulose. 23, 5, p. 3129-31
Ma, Q., Mohawk, D., Jahani, B., Wang, X., Chen, Y., Mahoney, A., Zhu, J. Y., Jiang, L. (2020) UV-Curable Cellulose Nanofiber-Reinforced Soy Protein Resins for 3D Printing and Conventional Molding. ACS Applied Polymer Materials, 2020, 2, 4666-4676.
Xu, C., Zhang Molino, B., Wang, X., Cheng, F., Xu, W., Molino, P., Bacher, M., Su, D., Rosenau, T., Willför, S., Wallace, G., (2018) 3D printing of nanocellulose hydrogel scaffolds with tunable mechanical strength towards wound healing application. Journal of Materials Chemistry B., 6 (43), 7066-7075.
Xu, W., Zhang Molino, B., Cheng, F., Molino, P., Yue, Z., Su, D., Wang, X., Willför, S., Xu, C., Wallace, G., (2019a) On low-concentration inks formulated by nanocellulose assisted with gelatin methacrylate (GelMA) for 3D printing toward wound healing application. ACS Applied Materials & Interfaces, 11 (9), 8838-8848.
Xu, W., Zhang, X., Yang, P., Långvik, O., Wang, X., Zhang, Y., Cheng, F., Österberg, M., Willför, S., Xu, C., (2019b) Surface engineered biomimetic inks based on UV cross-linkable wood biopolymers for 3D printing. ACS Applied Materials & Interfaces, 11 (13), 12389-12400.

## Claims

1. A hydrogel formulation, comprising methacrylated cellulose nanofibrils and water, wherein the methacrylated cellulose nanofibrils have a charge density of 1.2 mmol/g or lower, and wherein the methacrylated cellulose nanofibrils comprise cellulose nanofibrils obtained from cellulose fibers by treatments that introduce anionic charges.

2. The formulation according to claim 1, comprising a crosslinking agent, wherein the crosslinking agent comprises at least a photoinitiator, optionally with one additional Photo-/UV-crosslinking agent.

3. The formulation according to claim 1 or 2, comprising 0.1 to 3 %, in particular 0.5 to 2 %, of methacrylated cellulose nanofibrils, and optionally 0.1 to 5 %, in particular 0.5 to 3 %, of a crosslinking agent, calculated from the total weight of the formulation.

4. The formulation according to any of the preceding claims, wherein the methacrylated cellulose nanofibrils have a charge density of less than 1.2 mmol/g, preferably less than 0.8 mmol/g, in particular less than 0.5 mmol/g, suitably less than 0.4 mmol/g, such as less than 0.3 mmol/g.

5. The formulation according to any of the preceding claims, wherein the methacrylated cellulose nanofibrils are free of charge or essentially free of charge or free of additional charge by chemical or biochemical modification.

6. The formulation according to any of the preceding claims, wherein the methacrylated cellulose nanofibrils comprise cellulose nanofibrils obtained from cellulose fibers selected from the group of dissolving pulp, cotton fiber, bacterial cellulose fiber, and combinations thereof, by mechanical defibrillation, such as grinding.

7. The formulation according to any of claims 1 to 6, wherein the methacrylated cellulose nanofibrils comprise cellulose nanofibrils obtained from cellulose fibers by carboxymethylation, oxidation, TEMPO-oxidation, or acid treatment using sulfuric acid, hydrochloric acid, or phosphoric acid, or a combination thereof, preferably TEMPO-oxidation.

8. The formulation according to any of the preceding claims, wherein the methacrylated cellulose nanofibrils comprise cellulose nanofibrils obtained by removal of charge groups from cellulose nanofibrils having a charge of at least 1.2 mmol/g to produce cellulose nanofibrils having a charge density of less than 1.2 mmol/g.

9. The formulation according to any one of claims 2 to 8, further comprising at least one crosslinking agent selected from methacrylated biopolymers, monomeric acrylamides, methacrylic anhydride and combinations thereof, preferably from methacrylated biopolymers, wherein the biopolymers are preferably selected from gelatin, chitosan, alginate and wood polysaccharides, such as galactoglucomannan, xylan and xyloglucan, and combinations thereof.

10. The formulation according to claim 9, wherein the methacrylated biopolymer is gelatin methacrylate, methacrylated galactoglucomannan or a combination thereof.

11. The formulation according to any one of claims 2 to 10, wherein the crosslinking agent comprises acrylamide monomer, or methacrylated oligomer and polymer, or other crosslinking agents with alkenes, together with a photoinitiator.

12. The formulation according to any of the preceding claims, wherein the methacrylated cellulose nanofibrils comprise cellulose fibers subjected to oxidation in aqueous medium, followed by surface modification of hydroxyl groups on cellulose fibers with acrylic anhydrides, such as methacrylic anhydride or acrylic anhydride, and mechanical defibrillation of the methacrylated cellulose fibers to methacrylated cellulose nanofibrils.

13. The formulation according to any one of the preceding claims, wherein the methacrylated cellulose nanofibrils have a degree of substitution (DS) of methacrylates in the nanocellulose, wherein the degree of substitution of 0.005 to 0.5, preferably 0.01 to 0.03, more preferably about 0.02.

14. The formulation according to any one of the preceding claims, wherein the methacrylated cellulose nanofibrils comprise cellulose nanofibrils methacrylated on the hydroxy groups of cellulose fibrils.

15. The formulation according to any one of the preceding claims, further comprising biological material, wherein the biological material is selected from the group of vegetable and animal cells and tissue, in particular from the group of endothelial cells, fibroblasts, stem cells, chondrocytes and osteoblasts.

16. The formulation according to claim 15, which is essentially free from aggregates having a size greater than 1000 µm, in particular greater than 600 µm, for example greater than 500 µm.

17. A method of producing an ink formulation for 3D printing, comprising the steps of
- providing an aqueous gel of methacrylated cellulose nanofibrils,
- optionally providing at least one crosslinking agent, preferably a photoinitiator
- optionally providing an aqueous dispersion of biological material, and
- mixing the gel, the optional crosslinking agent and optionally the aqueous dispersion of biological material to form an ink composition,
wherein the methacrylated cellulose nanofibrils have a charge density of 1.2 mmol/g or lower and comprise methacrylated cellulose nanofibrils obtained from cellulose fibers subjected to oxidation in an aqueous medium.

18. The method according to claim 17, comprising the steps of
- providing an aqueous gel of methacrylated cellulose nanofibrils,
- providing a photoinitiator and optionally providing at least one additional crosslinking agent,
- optionally providing an aqueous dispersion of biological material, and
- mixing the gel, the photoinitiator and the optional additional crosslinking agent and optionally the aqueous dispersion of biological material to form an ink composition,
wherein the methacrylated cellulose nanofibrils have a charge density of 1.2 mmol/g or lower.

19. The method according to claim 17 or 18, wherein the aqueous gel of methacrylated cellulose nanofibrils is produced by subjecting cellulose nanofibrils to grafting methacryloyl groups to the fiber surface through reacting with either the hydroxyls or carboxyls within anionic cellulose fiber structure, wherein the grafting step comprises:
(a) modification with acrylic anhydrides, such as methacrylic anhydride; or
(b) modification with glycidyl methacrylate; or
(c) modification with aminoalkyl methacrylate, such as 2-aminoethyl methacrylate

20. The method according to claim 17 or 18, wherein the aqueous gel of methacrylated cellulose nanofibrils is produced by
- oxidizing cellulose fibers in an aqueous medium,
- subjecting the oxidized cellulose to solvent exchange from water to an organic solvent, preferably to dimethylformamide,
- surface modification of hydroxyl groups on cellulose fibers with acrylic anhydrides, such as methacrylic anhydride or acrylic anhydride, preferably with methacrylic anhydride,
- removal of the organic solvent and mechanical defibrillation of the methacrylated cellulose fibers in water to obtain the aqueous gel of methacrylated cellulose nanofibrils.

21. The method according to any of claims 17 to 20, wherein the ink formulation is produced by mixing a predetermined amount of a crosslinking agent with the aqueous gel of methacrylated cellulose nanofibrils containing 0.1 to 3 % of methacrylated cellulose nanofibrils to provide a modified hydrogel containing 0.1 to 3 % of methacrylated cellulose nanofibrils and about 0.1 to 5 %, of a crosslinking agent, calculated from the total weight of the formulation.

22. The method according to any of claims 17 to 21, wherein the methacrylated cellulose nanofibrils have a charge density of less than 1.2 mmol/g, in particular less than 1.0 mmol/g, suitably less than 0.9 mmol/g, such as less than 0.8 mmol/g, in particular the methacrylated cellulose nanofibrils are free of charge or essentially free of charge.

23. The method according to any one of claims 17 to 22, wherein the crosslinking agent is selected from monomeric acrylamide or methacrylated biopolymers and it is preferably added in the form of a powder or an aqueous suspension to the aqueous gel of methacrylated cellulose nanofibrils.

24. The method according to any of claims 17 to 23, comprising the steps of
- providing an aqueous suspension of biological material selected from the group of vegetable and animal cells and tissue, in particular from the group of endothelial cells, fibroblast, stem, chondrocyte and osteoblast,
- mixing the suspension with the aqueous hydrogel of methacrylated cellulose nanofibrils, and
- adding a Photo-/UV-crosslinking agent at a preselected step.

25. The method according to any of claims 17 to 24, wherein the crosslinking agent is selected from methacrylated biopolymers, the biopolymers preferably being selected from gelatin, chitosan, alginate and wood polysaccharides, such as galactoglucomannan, xylan and xyloglucan, and combinations thereof.

26. The use of a hydrogel formulation according to any of claims 1 to 16 in 3D printing, preferably by a direct ink writing-type printer.

27. The use of a hydrogel formulation according to any of claims 1 to 16 for producing, by printing,
- 3D structures, which are used for cell-matrix and cell-cell interaction studies, or
- 3D structures, which are used for drug screening or drug therapy studies, or
- 3D scaffolds suitable as cell culture platforms, for example for cell proliferation and cell differentiation, e.g. cancer cells, or as 3D scaffolds for disease modelling.

28. A hydrogel formulation according to any one of claims 1 to 16 for use in tissue and organ engineering and regenerative medicine.

29. 3D printed products comprising the formulation according to any one of claims 1 to 16.

## Patentansprüche

1. Hydrogelformulierung, umfassend methacrylierte Cellulose-Nanofibrillen und Wasser, wobei die methacrylierten Cellulose-Nanofibrillen eine Ladungsdichte von 1,2 mmol/g oder niedriger aufweisen, wobei die methacrylierten Cellulose-Nanofibrillen Cellulose-Nanofibrillen umfassen, die aus Cellulosefasern durch Behandlungen erhalten werden, die anionische Ladungen einführen.

2. Formulierung nach Anspruch 1, umfassend ein Vernetzungsmittel, wobei das Vernetzungsmittel mindestens einen Photoinitiator, optional mit einem zusätzlichen Photo-/UV-Vernetzungsmittel umfasst.

3. Formulierung nach Anspruch 1 oder 2, umfassend 0,1 bis 3 %, insbesondere 0,5 bis 2 %, methacrylierter Cellulose-Nanofibrillen und optional 0,1 bis 5 %, insbesondere 0,5 bis 3 %, eines Vernetzungsmittels, berechnet aus dem Gesamtgewicht der Formulierung.

4. Formulierung nach einem der vorstehenden Ansprüche, wobei die methacrylierten Cellulose-Nanofibrillen eine Ladungsdichte von weniger als 1,2 mmol/g, vorzugsweise weniger als 0,8 mmol/g, insbesondere weniger als 0,5 mmol/g, angemessen weniger als 0,4 mmol/g, wie zum Beispiel weniger als 0,3 mmol/g aufweisen.

5. Formulierung nach einem der vorstehenden Ansprüche, wobei die methacrylierten Cellulose-Nanofibrillen frei von Ladung oder im Wesentlichen frei von Ladung oder frei von zusätzlicher Ladung durch chemische oder biochemische Modifizierung sind.

6. Formulierung nach einem der vorstehenden Ansprüche, wobei die methacrylierten Cellulose-Nanofibrillen Cellulose-Nanofibrillen umfassen, die aus Cellulosefasern erhalten wurden, ausgewählt aus der Gruppe von Chemiezellstoff, Baumwollfasern, Bakteriencellulosefasern und Kombinationen davon, durch mechanische Defibrillierung, wie z. B. Mahlen.

7. Formulierung nach einem der Ansprüche 1 bis 6, wobei die methacrylierten Cellulose-Nanofibrillen Cellulose-Nanofibrillen umfassen, die aus Cellulosefasern durch Carboxymethylierung, Oxidation, TEMPO-Oxidation oder Säurebehandlung unter Verwendung von Schwefelsäure, Salzsäure oder Phosphorsäure oder einer Kombination davon, vorzugsweise TEMPO-Oxidation, erhalten wurden.

8. Formulierung nach einem der vorstehenden Ansprüche, wobei die methacrylierten Cellulose-Nanofibrillen Cellulose-Nanofibrillen umfassen, die durch Entfernen von Ladungsgruppen von Cellulose-Nanofibrillen, die eine Ladung von mindestens 1,2 mmol/g aufweisen zur Herstellung von Cellulose-Nanofibrillen mit einer Ladungsdichte von weniger als 1,2 mmol/g erhalten wurden.

9. Formulierung nach einem der Ansprüche 2 bis 8, weiter umfassend mindestens ein Vernetzungsmittel, ausgewählt aus methacrylierten Biopolymeren, monomeren Acrylamiden, Methacrylsäureanhydrid und Kombinationen davon, vorzugsweise aus methacrylierten Biopolymeren, wobei die Biopolymere vorzugsweise ausgewählt sind aus Gelatine, Chitosan, Alginat und Holzpolysacchariden wie Galactoglucomannan, Xylan und Xyloglucan sowie Kombinationen davon.

10. Formulierung nach Anspruch 9, wobei es sich bei dem methacrylierten Biopolymer um Gelatinemethacrylat, methacryliertes Galactoglucomannan oder eine Kombination davon handelt.

11. Formulierung nach einem der Ansprüche 2 bis 10, wobei das Vernetzungsmittel Acrylamidmonomer oder methacryliertes Oligomer und Polymer oder andere Vernetzungsmittel mit Alkenen zusammen mit einem Photoinitiator umfasst.

12. Formulierung nach einem der vorstehenden Ansprüche, wobei die methacrylierten Cellulose-Nanofibrillen Cellulosefasern umfassen, die in wässrigem Medium Oxidation unterzogen wurden, gefolgt von Oberflächenmodifizierung der Hydroxylgruppen auf Cellulosefasern mit Acrylanhydriden, wie Methacrylsäureanhydrid oder Acrylsäureanhydrid, und mechanischer Defibrillierung der methacrylierten Cellulosefasern zu methacrylierten Cellulose-Nanofibrillen.

13. Formulierung nach einem der vorstehenden Ansprüche, wobei die methacrylierten Cellulose-Nanofibrillen einen Substitutionsgrad (DS) von Methacrylaten in der Nanocellulose aufweisen, wobei der Substitutionsgrad 0,005 bis 0,5, vorzugsweise 0,01 bis 0,03, besonders bevorzugt etwa 0,02 beträgt.

14. Formulierung nach einem der vorstehenden Ansprüche, wobei die methacrylierten Cellulose-Nanofibrillen Cellulose-Nanofibrillen umfassen, die an den Hydroxygruppen der Cellulosefibrillen methacryliert sind.

15. Formulierung nach einem der vorstehenden Ansprüche, weiter umfassend biologisches Material, wobei das biologische Material ausgewählt ist aus der Gruppe pflanzlicher und tierischer Zellen und Gewebe, insbesondere aus der Gruppe von Endothelzellen, Fibroblasten, Stammzellen, Chondrozyten und Osteoblasten.

16. Formulierung nach Anspruch 15, die im Wesentlichen frei von Aggregaten mit einer Größe von mehr als 1000 µm, insbesondere größer als 600 µm, beispielsweise größer als 500 µm ist.

17. Verfahren zur Herstellung einer Tintenformulierung für 3D-Druck, das die folgenden Schritte umfasst
- Bereitstellen eines wässrigen Gels aus methacrylierten Cellulose-Nanofibrillen,
- optional Bereitstellen mindestens eines Vernetzungsmittels, vorzugsweise eines Photoinitiators,
- optional Bereitstellen einer wässrigen Dispersion von biologischem Material und
- Mischen des Gels, des optionalen Vernetzungsmittels und optional der wässrigen Dispersion von biologischem Material zum Bilden einer Tintenzusammensetzung,
wobei die methacrylierten Cellulose-Nanofibrillen eine Ladungsdichte von 1,2 mmol/g oder niedriger aufweisen und methacrylierte Cellulose-Nanofibrillen umfassen, die aus Cellulosefasern erhalten werden, die in einem wässrigen Medium Oxidation unterzogen wurden.

18. Verfahren nach Anspruch 17, das weiter die folgenden Schritte umfasst:
- Bereitstellen eines wässrigen Gels aus methacrylierten Cellulose-Nanofibrillen,
- Bereitstellen eines Photoinitiators und optional Bereitstellen mindestens eines weiteren Vernetzungsmittels,
- optional Bereitstellen einer wässrigen Dispersion von biologischem Material und
- Mischen des Gels, des Photoinitiators und des optionalen zusätzlichen Vernetzungsmittels und optional der wässrigen Dispersion von biologischem Material zum Bilden einer Tintenzusammensetzung,
wobei die methacrylierten Cellulose-Nanofibrillen eine Ladungsdichte von 1,2 mmol/g oder niedriger aufweisen.

19. Verfahren nach Anspruch 17 oder 18, wobei das wässrige Gel aus methacrylierten Cellulose-Nanofibrillen dadurch hergestellt wird, dass Cellulose-Nanofibrillen Aufpfropfen von Methacryloylgruppen auf die Faseroberfläche unterzogen werden durch Reagieren entweder mit den Hydroxyl- oder den Carboxylgruppen innerhalb der anionischen Cellulosefaserstruktur, wobei der Aufpfropfschritt Folgendes umfasst:
(a) Modifizierung mit Acrylanhydriden, wie beispielsweise Methacrylsäureanhydrid; oder
(b) Modifizierung mit Glycidylmethacrylat; oder
(c) Modifizierung mit Aminoalkylmethacrylat, wie z. B. 2-Aminoethylmethacrylat

20. Verfahren nach Anspruch 17 oder 18, wobei das wässrige Gel aus methacrylierten Cellulose-Nanofibrillen hergestellt wird durch
- Oxidieren von Cellulosefasern in einem wässrigen Medium,
- Unterziehen der oxidierten Cellulose einem Lösungsmittelaustausch von Wasser zu einem organischen Lösungsmittel, vorzugsweise zu Dimethylformamid,
- Oberflächenmodifizierung von Hydroxylgruppen auf Cellulosefasern mit Acrylanhydriden, wie z. B. Methacrylsäureanhydrid oder Acrylsäureanhydrid, vorzugsweise mit Methacrylsäureanhydrid,
- Entfernen des organischen Lösungsmittels und mechanische Defibrillierung der methacrylierten Cellulosefasern in Wasser, um das wässrige Gel aus methacrylierten Cellulose-Nanofibrillen zu erhalten.

21. Verfahren nach einem der Ansprüche 17 bis 20, wobei die Tintenformulierung hergestellt wird durch Mischen einer vorbestimmten Menge eines Vernetzungsmittels mit dem wässrigen Gel aus methacrylierten Cellulose-Nanofibrillen, das 0,1 bis 3 % methacrylierte Cellulose-Nanofibrillen enthält, um ein modifiziertes Hydrogel bereitzustellen, das 0,1 bis 3 % methacrylierte Cellulose-Nanofibrillen und etwa 0,1 bis 5 % eines Vernetzungsmittels enthält, berechnet aus dem Gesamtgewicht der Formulierung.

22. Verfahren nach einem der Ansprüche 17 bis 21, wobei die methacrylierten Cellulose-Nanofibrillen eine Ladungsdichte von weniger als 1,2 mmol/g, insbesondere weniger als 1,0 mmol/g, angemessen weniger als 0,9 mmol/g, wie zum Beispiel weniger als 0,8 mmol/g aufweisen, wobei die methacrylierten Cellulose-Nanofibrillen insbesondere frei oder im Wesentlichen frei von Ladung sind.

23. Verfahren nach einem der Ansprüche 17 bis 22, wobei das Vernetzungsmittel ausgewählt ist aus monomerem Acrylamid oder methacrylierten Biopolymeren und vorzugsweise in Form eines Pulvers oder einer wässrigen Suspension zu dem wässrigen Gel aus methacrylierten Cellulose-Nanofibrillen zugegeben wird.

24. Verfahren nach einem der Ansprüche 17 bis 23, das die folgenden Schritte umfasst
- Bereitstellen einer wässrigen Suspension von biologischem Material, ausgewählt aus der Gruppe der pflanzlichen und tierischen Zellen und Gewebe, insbesondere aus der Gruppe von Endothelzellen, Fibroblasten, Stammzellen, Chondrozyten und Osteoblasten,
- Mischen der Suspension mit dem wässrigen Hydrogel aus methacrylierten Cellulose-Nanofibrillen und
- Zugeben eines Photo-/UV-Vernetzungsmittel bei einem vorausgewählten Schritt.

25. Verfahren nach einem der Ansprüche 17 bis 24, wobei das Vernetzungsmittel ausgewählt ist aus methacrylierten Biopolymeren, wobei die Biopolymere vorzugsweise ausgewählt sind aus Gelatine, Chitosan, Alginat und Holzpolysacchariden wie Galactoglucomannan, Xylan und Xyloglucan sowie Kombinationen davon.

26. Verwendung einer Hydrogelformulierung nach einem der Ansprüche 1 bis 16 in 3D-Druck, vorzugsweise mittels eines Direct-Ink-Writing-Druckers.

27. Verwendung einer Hydrogelformulierung nach einem der Ansprüche 1 bis 16 zur Herstellung durch Drucken von
- 3D-Strukturen, die für Zell-Matrix- und Zell-Zell-Interaktionsstudien verwendet werden oder
- 3D-Strukturen, die für das Wirkstoff-Screening oder Studien zur Arzneimitteltherapie verwendet werden oder
- 3D-Gerüste, die als Zellkulturplattformen geeignet sind, beispielsweise für Zellproliferation und Zelldifferenzierung, z. B. von Krebszellen, oder als 3D-Gerüste für Krankheitsmodellierung.

28. Hydrogelformulierung nach einem der Ansprüche 1 bis 16 zur Verwendung in Gewebe- und Organengineering und der regenerativen Medizin.

29. 3D-gedruckte Produkte, die die Formulierung nach einem der Ansprüche 1 bis 16 umfassen.

## Revendications

1. Formulation d'hydrogel, comprenant des nanofibrilles de cellulose méthacrylatée et de l'eau, dans laquelle les nanofibrilles de cellulose méthacrylatée présentent une densité de charge de 1,2 mmol/g ou moins, et dans laquelle les nanofibrilles de cellulose méthacrylatées comprennent des nanofibrilles de cellulose obtenues à partir de fibres de cellulose par des traitements qui introduisent des charges anioniques.

2. Formulation selon la revendication 1, comprenant un agent de réticulation, dans laquelle l'agent de réticulation comprend au moins un photo-initiateur, facultativement avec un agent de photoréticulation/réticulation par UV.

3. Formulation selon la revendication 1 ou 2, comprenant 0,1 à 3 %, en particulier 0,5 à 2 %, de nanofibrilles de cellulose méthacrylatées et, facultativement, 0,1 à 5 %, en particulier 0,5 à 3 %, d'un agent de réticulation, calculé à partir du poids total de la formulation.

4. Formulation selon l'une quelconque des revendications précédentes, dans laquelle les nanofibrilles de cellulose méthacrylatée présentent une densité de charge inférieure à 1,2 mmol/g, de préférence inférieur à 0,8 mmol/g, en particulier inférieure à 0,5 mmol/g, de façon appropriée inférieure à 0,4 mmol/g, par exemple, inférieure à 0,3 mmol/g.

5. Formulation selon l'une quelconque des revendications précédentes, dans laquelle les nanofibrilles de cellulose méthacrylatée sont exemptes de charges ou essentiellement exemptes de charges ou exemptes de charges supplémentaires par modification chimique ou biochimique.

6. Formulation selon l'une quelconque des revendications précédentes, dans laquelle les nanofibrilles de cellulose méthacrylatées comprennent des nanofibrilles de cellulose obtenues à partir de fibres de cellulose sélectionnées dans le groupe constitué par une pâte à dissoudre, une fibre de coton, une fibre de cellulose bactérienne et des combinaisons de celles-ci, par défibrillation mécanique, telle que le broyage.

7. Formulation selon l'une quelconque des revendications 1 à 6, dans laquelle les nanofibrilles de cellulose méthacrylatées comprennent des nanofibrilles de cellulose obtenues à partir de fibres de cellulose par carboxyméthylation, oxydation, oxydation TEMPO ou traitement acide utilisant de l'acide sulfurique, de l'acide chlorhydrique ou de l'acide phosphorique, ou une combinaison de ceux-ci, de préférence par oxydation TEMPO.

8. Formulation selon l'une quelconque des revendications précédentes, dans laquelle les nanofibrilles de cellulose méthacrylatées comprennent des nanofibrilles de cellulose obtenues par élimination de groupes chargés à partir de nanofibrilles de cellulose présentant une charge d'au moins 1,2 mmol/g pour produire des nanofibrilles de cellulose présentant une densité de charge inférieure à 1,2 mmol/g.

9. Formulation selon l'une quelconque des revendications 2 à 8, comprenant en outre au moins un agent de réticulation choisi parmi les biopolymères méthacrylatés, les acrylamides monomères, l'anhydride méthacrylique et des combinaisons de ceux-ci, de préférence parmi les biopolymères méthacrylatés, dans laquelle les biopolymères sont, de préférence, choisis parmi la gélatine, le chitosane, l'alginate et les polysaccharides du bois, tels que le galactoglucomannane, le xylane et le xyloglucane, et des combinaisons de ceux-ci.

10. Formulation selon la revendication 9, dans laquelle le biopolymère méthacrylaté est le méthacrylate de gélatine, le galactoglucomannane méthacrylaté ou une combinaison de ceux-ci.

11. Formulation selon l'une quelconque des revendications 2 à 10, dans laquelle l'agent de réticulation comprend un monomère d'acrylamide, ou un oligomère et un polymère méthacrylaté, ou d'autres agents de réticulation avec des alcènes, ainsi qu'un photoinitiateur.

12. Formulation selon l'une quelconque des revendications précédentes, dans laquelle les nanofibrilles de cellulose méthacrylatée comprennent des fibres de cellulose soumises à une oxydation en milieu aqueux, suivie d'une modification de surface des groupes hydroxyles sur les fibres de cellulose avec des anhydrides acryliques, tels que l'anhydride méthacrylique ou l'anhydride acrylique, et d'une défibrillation mécanique des fibres de cellulose méthacrylatée en nanofibrilles de cellulose méthacrylatée.

13. Formulation selon l'une quelconque des revendications précédentes, dans laquelle les nanofibrilles de cellulose méthacrylatées présentent un degré de substitution (DS) de méthacrylates dans la nanocellulose, dans laquelle le degré de substitution est 0,005 à 0,5, de préférence 0,01 à 0,03, plus préférentiellement d'environ 0,02.

14. Formulation selon l'une quelconque des revendications précédentes, dans laquelle les nanofibrilles de cellulose méthacrylatées comprennent des nanofibrilles de cellulose méthacrylatées sur les groupes hydroxyles de fibrilles de cellulose.

15. Formulation selon l'une quelconque des revendications précédentes, comprenant en outre un matériel biologique, dans laquelle le matériel biologique est choisi dans le groupe constitué par des cellules végétales et animales et des tissus végétaux et animaux, et, en particulier, dans le groupe constitué par des cellules endothéliales, des fibroblastes, des cellules souches, des chondrocytes et des ostéoblastes.

16. Formulation selon la revendication 15, qui est essentiellement exempte d'agrégats présentant une taille supérieure à 1 000 µm, en particulier supérieure à 600 µm, par exemple supérieure à 500 µm.

17. Procédé de production d'une formulation d'encre pour une impression 3D, comprenant les étapes de :
- fourniture d'un gel aqueux de nanofibrilles de cellulose méthacrylatée,
- fourniture facultative d'au moins un agent de réticulation, de préférence un photoinitiateur
- fourniture facultative d'une dispersion aqueuse de matériel biologique, et
- mélange du gel, de l'agent de réticulation facultatif et, facultativement, de la dispersion aqueuse de matière biologique pour former une composition d'encre,
dans lequel les nanofibrilles de cellulose méthacrylatée présentent une densité de charge de 1,2 mmol/g ou moins et comprennent des nanofibrilles de cellulose méthacrylatées obtenues à partir de fibres de cellulose soumises à une oxydation en milieu aqueux.

18. Procédé selon la revendication 17, comprenant les étapes de :
- fourniture d'un gel aqueux de nanofibrilles de cellulose méthacrylatée,
- fourniture d'un photoinitiateur et, facultativement, d'au moins un agent de réticulation supplémentaire,
- fourniture facultative d'une dispersion aqueuse de matériel biologique, et
- mélange du gel, du photo-initiateur et de l'agent de réticulation supplémentaire facultatif et, facultativement, de la dispersion aqueuse de matière biologique pour former une composition d'encre,
dans lequel les nanofibrilles de cellulose méthacrylatée présentent une densité de charge de 1,2 mmol/g ou moins.

19. Procédé selon la revendication 17 ou 18, dans lequel le gel aqueux de nanofibrilles de cellulose méthacrylatées est produit en soumettant des nanofibrilles de cellulose à la greffe de groupes méthacryloyle à la surface de la fibre par réaction avec les groupes soit hydroxyles, soit carboxyles de la structure anionique de la fibre de cellulose, dans lequel l'étape de greffage comprend :
a) la modification par des anhydrides acryliques, tels que l'anhydride méthacrylique ; ou
b) la modification par un méthacrylate de glycidyle ; ou
c) la modification par un méthacrylate d'aminoalkyle, tel que le méthacrylate de 2-aminoéthyle

20. Procédé selon la revendication 17 ou 18, dans lequel le gel aqueux de nanofibrilles de cellulose méthacrylatée est produit par
- oxydation de fibres de cellulose en milieu aqueux,
- soumission de la cellulose oxydée à un échange de solvant de l'eau à un solvant organique, de préférence au diméthylformamide,
- modification de surface des groupes hydroxyle sur les fibres de cellulose avec des anhydrides acryliques, tels que l'anhydride méthacrylique ou l'anhydride acrylique, de préférence avec l'anhydride méthacrylique,
- élimination du solvant organique et défibrillation mécanique des fibres de cellulose méthacrylatée dans l'eau pour obtenir le gel aqueux de nanofibrilles de cellulose méthacrylatée.

21. Procédé selon l'une quelconque des revendications 17 à 20, dans lequel la formulation d'encre est produite en mélangeant une quantité prédéterminée d'un agent de réticulation avec le gel aqueux de nanofibrilles de cellulose méthacrylatée contenant 0,1 à 3 % de nanofibrilles de cellulose méthacrylatées pour obtenir un hydrogel modifié contenant 0,1 à 3 % de nanofibrilles de cellulose méthacrylatée et environ 0,1 à 5 %, d'un agent de réticulation, calculé à partir du poids total de la formulation.

22. Procédé selon l'une quelconque des revendications 17 à 21, dans lequel les nanofibrilles de cellulose méthacrylatées présentent une densité de charge inférieure à 1,2 mmol/g, en particulier inférieure à 1,0 mmol/g, de façon appropriée inférieure à 0,9 mmol/g, par exemple, inférieure à 0,8 mmol/g, en particulier, les nanofibrilles de cellulose méthacrylatée sont exemptes de charges ou essentiellement exemptes de charges.

23. Procédé selon l'une quelconque des revendications 17 à 22, dans lequel l'agent de réticulation est choisi parmi l'acrylamide monomère ou des biopolymères méthacrylatés et est, de préférence, ajouté sous la forme d'une poudre ou d'une suspension aqueuse au gel aqueux de nanofibrilles de cellulose méthacrylatée.

24. Procédé selon l'une quelconque des revendications 17 à 23, comprenant les étapes de
- fourniture d'une suspension aqueuse de matériel biologique sélectionné dans le groupe constitué par les cellules végétales et animales et les tissus végétaux et animaux, en particulier dans le groupe constitué par des cellules endothéliales, des fibroblastes, des cellules souches, des chondrocytes et des ostéoblastes,
- mélange de la suspension avec l'hydrogel aqueux de nanofibrilles de cellulose méthacrylatée, et
- ajoute d'un agent de photoréticulation/réticulation par UV à une étape présélectionnée.

25. Procédé selon l'une quelconque des revendications 17 à 24, dans lequel l'agent de réticulation est choisi parmi des biopolymères méthacrylatés, les biopolymères étant, de préférence, choisis parmi la gélatine, le chitosane, l'alginate et les polysaccharides du bois, tels que le galactoglucomannane, le xylane et le xyloglucane, et des combinaisons de ceux-ci.

26. Utilisation d'une formulation d'hydrogel selon l'une quelconque des revendications 1 à 16 dans une impression 3D, de préférence par une imprimante à écriture directe à l'encre.

27. Utilisation d'une formulation d'hydrogel selon l'une quelconque des revendications 1 à 16 pour produire, par impression,
- des structures 3D, qui sont utilisées pour des études d'interaction cellule-matrice et cellule-cellule, ou
- des structures 3D, qui sont utilisées pour des études de criblage de médicaments ou de thérapie médicamenteuse, ou
- des échafaudages 3D appropriés en tant que plateformes de culture cellulaire, par exemple pour une prolifération cellulaire et une différenciation cellulaire, par exemple des cellules cancéreuses, ou en tant qu'échafaudages 3D pour une modélisation des maladies.

28. Formulation d'hydrogel selon l'une quelconque des revendications 1 à 16 pour une utilisation en ingénierie tissulaire et organique et en médecine régénérative.

29. Produits imprimés en 3D comprenant la formulation selon l'une quelconque des revendications 1 à 16.
